# EUROPEAN PATENT APPLICATION

(11) **EP 1 486 483 A1**
(43) Date of publication of application: **15.12.2004**
(21) Application number: 03707030.7
(22) Date of filing: 25.02.2003
(51) Int. Cl.: C07C 235/38, C07C 255/13, C07C 255/63, C07C 323/37, C07C 323/60, C07C 323/62, C07C 327/44

(54) **N-BIPHENYLAMIDES AND FUNGICIDAL COMPOSITIONS CONTAINING THE SAME**

(30) Priority: 15.03.2002 JP 2002071627
(71) Applicant: Sumitomo Chemical Company, Limited, Osaka-shi Osaka 541-8550 (JP)
(72) Inventor: SAKAGUCHI, Hiroshi, Toyonaka-shi, Osaka 561-0802 (JP)
(74) Representative: Henkel, Feiler & Hänzel
(86) International application number: PCT/JP2003/002015
(87) International publication number: WO 2003/078382

(57) **Abstract**

A N-biphenylamide compound of the formula (1): 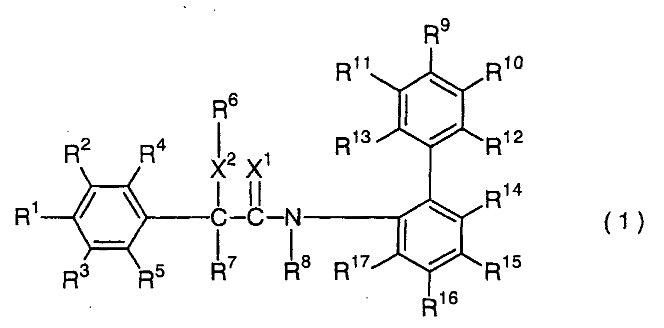 wherein R¹, R², R³, R⁴ and R⁵ independently represent a hydrogen atom, halogen atom or the like, R⁶ represents C1-C6 alkyl and the like, R⁷ represents a hydrogen atom or C1-C3 alkyl,
R⁸ represents a hydrogen atom or C1-C3 alkyl,
R⁹ and R¹⁰ independently represents a halogen atom, C1-C6 alkyl, C1-C6 alkoxy or the like,
R¹¹, R¹² and R¹³ independently represent a hydrogen atom, halogen atom or the like, R¹⁴, R¹⁵, R¹⁶ and R¹⁷ independently is a hydrogen atom, halogen atom or the like, X¹ represents an oxygen atom or sulfur atom, X² represents an oxygen atom or sulfur atom;
has excellent effect aginst plant diseases.

## Description

### Technical Field of the Invention

The present invention relates to N-biphenylamide compounds and fungicidal compositions containing the same.

### Background Art

Some of N-biphenylamide compounds are disclosed in International Patent Application published under the Patent Cooperation Treaty WO97/08148, European Patent Published Application 0545099 and the like.

The object of the present invention is to provide a novel N-biphenylamide compound having excellent controlling activity against plant diseases.

### Disclosure of the Invention

The present inventor has earnestly studied, and found that the N-biphenylamide compound of the formula (1) has excellent plant diseases controlling effect to complete the present invention.

Namely, the present invention provides the N-biphenylamide compound (hereinafter, referred to as the present compound) of the following formula (1): 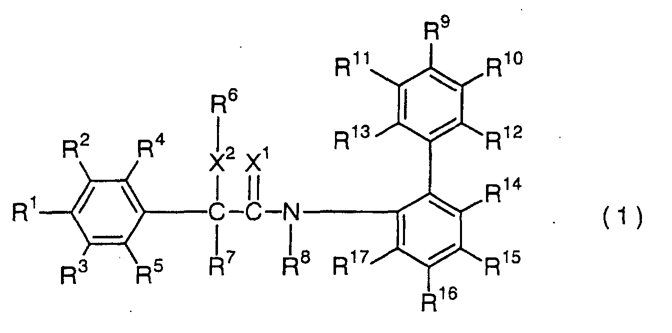 wherein R¹, R², R³, R⁴ and R⁵ independently represent a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkoxy, tri(C1-C6 alkyl)silyl or cyano,
R⁶ represents C1-C6 alkyl, C1-C6 haloalkyl, C3-C6 alkenyl, C3-C6 haloalkenyl, C3-C6 alkynyl, C3-C6 haloalkynyl, (C1-C6 alkyl)carbonyl, (C1-C6 haloalkyl)carbonyl, (C2-C6 alkenyl)carbonyl, (C2-C6 haloalkenyl)carbonyl, (C2-C6 alkynyl)carbonyl or (C2-C6 haloalkynyl)carbonyl,
R⁷ represents a hydrogen atom or C1-C3 alkyl,
R⁸ represents a hydrogen atom or C1-C3 alkyl,
R⁹ and R¹⁰ independently represents a halogen atom, C1-C6 alkyl, C1-C6 haloalkyl, C2-C6 alkenyl, C2-C6 haloalkenyl, C2-C6 alkynyl, C2-C6 haloalkynyl, cyano C1-C6 alkyl, C1-C6 alkoxy, C1-C6 haloalkoxy, C3-C6 alkenyloxy, C3-C6 haloalkenyloxy, C3-C6 alkynyloxy, C3-C6 haloalkynyloxy, cyano C1-C6 alkoxy, C1-C6 alkylthio, C1-C6 haloalkylthio, C3-C6 alkenylthio, C3-C6 alkynylthio, C3-C6 cycloalkyl, C3-C6 cycloalkoxy, (C3-C6 cycloalkyl) C1-C3 alkoxy, C2-C6 alkoxyalkyl, tri(C1-C6 alkyl)silyl or cyano,
R¹¹, R¹² and R¹³ independently represent a hydrogen atom, halogen atom or C1-C3 alkyl,
R¹⁴, R¹⁵, R¹⁶ and R¹⁷ independently is a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkyloxy, tri(C1-C6 alkyl)silyl or cyano,
X¹ represents an oxygen atom or sulfur atom,
X² represents an oxygen atom or sulfur atom;
the fungicidal composition comprising the present compound as an active ingredients; and the method for controlling plant diseases comprising applying the present compound to the plant or the soil.

In the present invention, a specific example of each substituent in the formula (1) is shown in the following.

In R¹, R², R³, R⁴ and R⁵;
the halogen atom includes, for example, a fluorine atom, chlorine atom and bromine atom;
the C1-C6 alkyl includes, for example, methyl, ethyl, propyl, isopropyl, butyl, isobutyl, *sec*-butyl, *tert*-butyl, pentyl, isopentyl and hexyl;
the C1-C6 haloalkyl includes, for example, fluoromethyl, difluoromethyl, trifluoromethyl, chlorodifluoromethyl, chlorofluoromethyl, bromodifluoromethyl, trichloromethyl, dichlorobromomethyl, 1,1,2,2,2-pentafluoroethyl, 2,2,2-trifluoroethyl, 2,2-difluoroethyl, 2-fluoroethyl and 6,6,6-trifluorohexyl;
the C2-C6 alkenyl includes, for example, vinyl, 2-propenyl, 1-methyl-2-propenyl, 2-methyl-2-propenyl, 2-butenyl, 3-butenyl, 2-hexenyl and 5-hexenyl;
the C2-C6 haloalkenyl includes, for example, 1-chlorovinyl, 2-chlorovinyl, 2,2-dichlorovinyl, 2,2-difluorovinyl, 1,2-dichlorovinyl, 3,3-dichloro-2-propenyl and 3,3-difluoro-2-propenyl;
the C2-C6 alkynyl includes, for example, ethynyl, 3-butynyl, 3-hexynyl and 5-hexynyl;
the C2-C6 haloalkynyl includes, for example, 2-chloroethynyl, 2-bromoethynyl, 3-chloro-2-propynyl, 3-bromo-2-propynyl and 6-chloro-5-hexynyl;
the C1-C6 alkoxy, for example, methoxy, ethoxy, propoxy, isopropoxy, butoxy, isobutoxy, sec-butoxy, *tert*-butoxy, pentyloxy, isopentyloxy and hexyloxy;
the C1-C6 haloalkoxy includes, for example, trifluoromethoxy, difluoromethoxy, bromodifluoromethoxy, chlorodifluoromethoxy, fluoromethoxy, 2,2,2-trifluoroethoxy, 1,1,2,2-tetrafluoroethoxy, 5-chloropentyloxy, 4-fluoroisopentyloxy and 2,2-dichlorohexyloxy;
the C3-C6 alkenyloxy includes, for example, 2-propenyloxy, 1-methyl-2-propenyloxy, 2-methyl-2-propenyloxy, 2-butenyloxy, 3-butenyloxy, 2-hexenyloxy and 5-hexenyloxy;
the C3-C6 haloalkenyloxy includes, for example, 3,3-dichloro-2-propenyloxy, 3,3-difluoro-2-propenyloxy, 3,3-dibromo-2-propenyloxy, 2,3-dichloropropenyloxy, 6-fluoro-2-hexenyloxy and 2,2-dichloro-5-hexenyloxy;
the C3-C6 alkynyloxy includes, for example, 2-propynyloxy, 1-methyl-2-propynyloxy, 2-butynyloxy, 3-butynyloxy, 2-hexynyloxy and 5-hexynyloxy;
the C3-C6 haloalkynyloxy includes, for example, 3-chloro-2-propynyloxy, 3-bromo-2-propynyloxy, 3-iodo-2-propynyloxy and 6-chloro-5-hexynyloxy;
the C1-C6 alkylthio includes, for example, methylthio, ethylthio, propylthio, isopropylthio, butylthio, isobutylthio, *sec*-butylthio, *tert*-butylthio, pentylthio, isopentylthio and hexylthio;
the C1-C6 haloalkylthio includes, for example, trifluoromethylthio, difluoromethylthio, bromodifluoromethylthio, chlorodifluoromethylthio, fluoromethylthio, 2,2,2-trifluoroethylthio, 1,1,2,2-tetrafluoroethylthio, 5-chloropentylthio, 4-fluoroisopentylthio and 2,2-dichlorohexylthio;
the C3-C6 cycloalkyl includes, for example, cyclopropyl, cyclopentyl and cyclohexyl;
the C3-C6 cycloalkoxy includes, for example, cyclopropoxy, cyclobutoxy, cyclopentoxy and cyclohexyloxy;
the tri(C1-C6 alkyl)silyl includes, for example, trimethylsilyl, triethylsilyl, tributylsilyl and *tert*-butyldimethylsilyl.

The phenyl substituted with R¹, R², R³, R⁴ and R⁵ includes, for example, the phenyl wherein R¹, R² and R³ are independently a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkoxy, tri(C1-C6 alkyl)silyl or cyano, and R⁴ and R⁵ are hydrogen atoms;
the phenyl wherein R¹ and R² are independently a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkoxy, tri(C1-C6 alkyl)silyl or cyano, and R³, R⁴ and R⁵ are hydrogen atoms;
the phenyl wherein R¹ is a chlorine atom, R² is a fluorine atom, and R³, R⁴ and R⁵ are hydrogen atoms;
the phenyl wherein R¹ and R² are chlorine atoms, and R³, R⁴ and R⁵ are hydrogen atoms;
the phenyl wherein R¹ is a chlorine atom, R² is a bromine atom, and R³, R⁴ and R⁵ are hydrogen atoms;
the phenyl wherein R¹ is a chlorine atom, R² is methyl, and R³, R⁴ and R⁵ are hydrogen atoms;
the phenyl wherein R¹ is a chlorine atom, R² is methoxy, and R³, R⁴ and R⁵ are hydrogen atoms;
the phenyl wherein R¹ is a bromine atom, R² is a fluorine atom, and R³, R⁴ and R⁵ are hydrogen atoms;
the phenyl wherein R¹ is a bromine atom, R² is a chlorine atom, and R³, R⁴ and R⁵ are hydrogen atoms;
the phenyl wherein R¹ and R² are bromine atoms, and R³, R⁴ and R⁵ are hydrogen atoms;
the phenyl wherein R¹ is a bromine atom, R² is methyl, and R³, R⁴ and R⁵ are hydrogen atoms;
the phenyl wherein R¹ is a bromine atom, R² is methoxy, and R³, R⁴ and R⁵ are hydrogen atoms;
the phenyl wherein R¹ is methyl, R² is a fluorine atom, and R³, R⁴ and R⁵ are hydrogen atoms;
the phenyl wherein R¹ is methyl, R² is a chlorine atom, and R³, R4 and R⁵ are hydrogen atoms;
the phenyl wherein R¹ is methyl, R² is a bromine atom, and R³, R⁴ and R⁵ are hydrogen atoms;
the phenyl wherein R¹ and R² are methyl, and R³, R⁴ and R⁵ are hydrogen atoms;
the phenyl wherein R¹ is methyl, R² is methoxy, and R³, R⁴ and R⁵ are hydrogen atoms;
the phenyl wherein R¹ is a fluorine atom, and R², R³, R⁴ and R⁵ are hydrogen atoms;
the phenyl wherein R¹ is a chlorine atom, and R², R³, R⁴ and R⁵ are hydrogen atoms;
the phenyl wherein R¹ is a bromine atom, and R², R³, R⁴ and R⁵ are hydrogen atoms;
the phenyl wherein R¹ is a iodine atom, and R², R³, R⁴ and R⁵ are hydrogen atoms;
the phenyl wherein R¹ is methyl, and R², R³, R⁴ and R⁵ are hydrogen atoms;
the phenyl wherein R¹ is ethyl, and R², R³, R⁴ and R⁵ are hydrogen atoms;
the phenyl wherein R¹ is propyl, and R², R³, R⁴ and R⁵ are hydrogen atoms;
the phenyl wherein R¹ is isopropyl, and R², R³, R⁴ and R⁵ are hydrogen atoms;
the phenyl wherein R¹ is fluoromethyl, and R², R³, R⁴ and R⁵ are hydrogen atoms;
the phenyl wherein R¹ is difluoromethyl, and R², R³, R⁴ and R⁵ are hydrogen atoms;
the phenyl wherein R¹ is trifluoromethyl, and R², R³, R⁴ and R⁵ are hydrogen atoms;
the phenyl wherein R¹ is 2-fluoroethyl, and R², R³, R⁴ and R⁵ are hydrogen atoms;
the phenyl wherein R¹ is 2,2-difluoroethyl, and R², R³, R⁴ and R⁵ are hydrogen atoms;
the phenyl wherein R¹ is 2,2,2-trifluoroethyl, and R², R³, R⁴ and R⁵ are hydrogen atoms;
the phenyl wherein R¹ is 1,2,2,2- tetrafluoroethyl, and R², R³, R⁴ and R⁵ are hydrogen atoms;
the phenyl wherein R¹ is pentafluoroethyl, and R², R³, R⁴ and R⁵ are hydrogen atoms;
the phenyl wherein R¹ is vinyl, and R², R³, R⁴ and R⁵ are hydrogen atoms;
the phenyl wherein R¹ is 1-methylvinyl, and R², R³, R⁴ and R⁵ are hydrogen atoms;
the phenyl wherein R¹ is 1-propenyl, and R², R³, R⁴ and R⁵ are hydrogen atoms;
the phenyl wherein R¹ is ethynyl, and R², R³, R⁴ and R⁵ are hydrogen atoms;
the phenyl wherein R¹ is 1-propynyl, and R², R³, R⁴ and R⁵ are hydrogen atoms;
the phenyl wherein R¹ is methoxy, and R², R³, R⁴ and R⁵ are hydrogen atoms;
the phenyl wherein R¹ is ethoxy, and R², R³, R⁴ and R⁵ are hydrogen atoms;
the phenyl wherein R¹ is propoxy, and R², R³, R⁴ and R⁵ are hydrogen atoms;
the phenyl wherein R¹ is isopropoxy, and R², R³, R⁴ and R⁵ are hydrogen atoms;
the phenyl wherein R¹ is fluoromethoxy, and R², R³, R⁴ and R⁵ are hydrogen atoms;
the phenyl wherein R¹ is difluoroethoxy, and R², R³, R⁴ and R⁵ are hydrogen atoms;
the phenyl wherein R¹ is trifluoroethoxy, and R², R³, R⁴ and R⁵ are hydrogen atoms;
the phenyl wherein R¹ is methylthio, and R², R³, R⁴ and R⁵ are hydrogen atoms;
the phenyl wherein R¹ is ethylthio, and R², R³, R⁴ and R⁵ are hydrogen atoms;
the phenyl wherein R¹ is propylthio, and R², R³, R⁴ and R⁵ are hydrogen atoms;
the phenyl wherein R¹ is isopropylthio, and R², R³, R⁴ and R⁵ are hydrogen atoms;
the phenyl wherein R¹ is fluoromethylthio, and R², R³, R⁴ and R⁵ are hydrogen atoms;
the phenyl wherein R¹ is difluoromethylthio, and R², R³, R⁴ and R⁵ are hydrogen atoms;
the phenyl wherein R¹ is trifluoromethylthio, and R², R³, R⁴ and R⁵ are hydrogen atoms;
the phenyl wherein R¹ is cyclopropyl, and R², R³, R⁴ and R⁵ are hydrogen atoms;
the phenyl wherein R² is a fluorine atom, and R¹, R³, R⁴ and R⁵ are hydrogen atoms;
the phenyl wherein R² is a chlorine atom, and R¹, R³, R⁴ and R⁵ are hydrogen atoms;
the phenyl wherein R² is a bromine atom, and R¹, R³, R⁴ and R⁵ are hydrogen atoms;
the phenyl wherein R² is methyl, and R¹, R³, R⁴ and R⁵ are hydrogen atoms;
the phenyl wherein R² is methoxy, and R¹, R³, R⁴ and R⁵ are hydrogen atoms; and
the phenyl wherein R¹, R², R³, R⁴ and R⁵ are hydrogen atoms.

In R⁶;
the C1-C6 alkyl includes, for example, methyl, ethyl, propyl, isopropyl, butyl, isobutyl, *sec*-butyl, *tert*-butyl, pentyl, isopentyl and hexyl;
the C1-C6 haloalkyl includes, for example, fluoromethyl, difluoromethyl, bromodifluoromethyl, chlorofluoromethyl, bromodifluoromethyl, 2,2,2-trifluoroethyl, 2,2-difluoroethyl, 2-fluoroethyl and 6,6,6-trifluorohexyl;
the C3-C6 alkenyl includes, for example; 2-propenyl, 1-methyl-2-propenyl, 2-methyl-2-propenyl, 2-butenyl; 3-butenyl, 2-hexenyl and 5-hexenyl;
the C3-C6 haloalkenyl includes, for example, 3,3-dichloro-2-propenyl and 3,3-difluoro-2-propenyl;
the C3-C6 alkynyl includes, for example, a 2-propynyl, 1-methyl-2-propynyl, 2-butynyl, 3-butynyl, 2-hexynyl and 5-hexynyl;
the C3-C6 haloalkynyl includes, for example, 3-chloro-2-propynyl, 3-bromo-2-propynyl and 6-chloro-5-hexynyl;
the (C1-C6 alkyl)carbonyl includes, for example, acetyl, propionyl, butyryl, isobutyryl, valeryl, isovaleryl, 2-methylbutanoyl, pivaloyl, hexanoyl, 4-methylpentanoyl and heptanoyl;
the (C1-C6 haloalkyl)carbonyl includes, for example, fluoroacetyl, difluoroacetyl, bromodifluoroacetyl, chlorofluoroacetyl, bromodifluoroacetyl, 3,3,3-trifluoropropanoyl, 3,3-difluoropropanoyl, 3-fluoropropanoyl and 7,7,7-trifluoroheptanoyl;
the (C2-C6 alkenyl)carbonyl includes, for example, acryloyl, 3-butenoyl, 2-methylbutenoyl, 3-methylbutenoyl, 3-pentenoyl, 4-pentenoyl, 3-heptenoyl and 6-heptenoyl;
the (C2-C6 haloalkenyl)carbonyl includes, for example, 4,4-dichloro-3-butenoyl and 4,4-difluoro-3-butenoyl;
the (C2-C6 alkynyl)carbonyl includes, for example, propioloyl, 3-butynoyl, 2-methyl-3-butynoyl, 3-pentynoyl, 4-pentynoyl, 3-heptynoyl and 6-heptynoyl;
the (C2-C6 haloalkynyl)carbonyl includes, for example, 4-chloro-3-butynoyl, 4-bromo-3-butynoyl and 7-chloro-6-heptynoyl.

In R⁷, the C1-C3 alkyl includes, for example, methyl, ethyl and propyl.

In R⁸, the C1-C3 alkyl includes, for example, methyl, ethyl and propyl.

In R⁹ and R¹⁰;
the halogen atom includes, for example, a fluorine atom and chlorine atom;
the C1-C6 alkyl includes, for example, methyl, ethyl, propyl, isopropyl, butyl, isobutyl, *sec*-butyl, *tert*-butyl, pentyl, isopentyl and hexyl;
the C1-C6 haloalkyl includes, for example, fluoromethyl, difluoromethyl, trifluoromethyl, trichloromethyl, 2,2,2-trifluoroethyl, 2,2-difluoroethyl and 2-fluoroethyl;
the C2-C6 alkenyl includes, for example, vinyl, 2-propenyl, 1-methyl-2-propenyl, 2-methyl-2-propenyl, 2-butenyl, 3-butenyl, 2-hexenyl and 5-hexenyl;
the C2-C6 haloalkenyl includes, for example, 1-chlorovinyl, 2-chlorovinyl, 2,2-dichlorovinyl, 2,2-difluorovinyl, 1,2-dichlorovinyl, 3,3-dichloro-2-propenyl and 3,3-difluoro-2-propenyl;
the C2-C6 alkynyl includes, for example, ethynyl, 3-butynyl and 5-hexynyl;
the C2-C6 haloalkynyl includes, for example, 2-chloroethynyl, 2-bromoethynyl and 6-chloro-5-hexynyl;
the C1-C6 alkoxy, for example, methoxy, ethoxy, propoxy, isopropoxy, butoxy, isobutoxy, *sec*-butoxy, *tert*-butoxy, pentyloxy, isopentyloxy and hexyloxy;
the C1-C6 haloalkoxy includes, for example, trifluoromethoxy, difluoromethoxy, bromodifluoromethoxy, chlorodifluoromethoxy, fluoromethoxy, 2,2,2-trifluoroethoxy, 1,1,2,2-tetrafluoroethoxy, 5-chloropentyloxy, 4-fluoroisopentyloxy, 2,2-dichlorohexyloxy, trichloromethoxy, 2,2-difluoroethoxy and 2-fluoroethoxy;
the C3-C6 alkenyloxy includes, for example, 2-propenyloxy, 1-methyl-2-propenyloxy, 2-methyl-2-propenyloxy, 2-butenyloxy, 3-butenyloxy, 2-hexenyloxy and 5-hexenyloxy;
the C3-C6 haloalkenyloxy includes, for example, 3,3-difluoro-2-propenyloxy, 3,3-dichloro-2-propenyloxy, 3,3-dibromo-2-propenyloxy, 2,3-dichloro-2-propenyloxy, 6-fluoro-2-hexenyloxy and 2,2-dichloro-5-hexenyloxy;
the C3-C6 alkynyloxy includes, for example, a 2-propynyloxy, 1-methyl-2-propynyloxy, 2-butynyloxy, 3-butynyloxy, 2-hexynyloxy and 5-hexynyloxy;
the C3-C6 haloalkynyloxy includes, for example, 3-chloro-2-propynyloxy, 3-bromo-2-propynyloxy, 3-iodo-2-propynyloxy and 6-chloro-5-hexynyloxy;
the cyano C1-C6 alkoxy includes, for example, cyanomethoxy, 1-cyanoethoxy, 2-cyanoethoxy, 2-methyl-2-cyanoethoxy, 1-cyanopropoxy and 2-cyanopropoxy;
the C1-C6 alkylthio includes, for example, methylthio, ethylthio, propylthio, isopropylthio, butylthio, isobutylthio, *sec*-butylthio, *tert*-butylthio, pentylthio, isopentylthio and hexylthio;
the C1-C6 haloalkylthio includes, for example, trifluoromethylthio, difluoromethylthio, bromodifluoromethylthio, chlorodifluoromethylthio, fluoromethylthio, 2,2,2-trifluoroethylthio, 1,1,2,2-tetrafluoroethylthio, 5-chloropentylthio, 4-fluoroisopentylthio and 2,2-dichlorohexylthio;
the C3-C6 cycloalkyl includes, for example, cyclopropyl, cyclobutyl, cyclopentyl and cyclohexyl;
the C3-C6 cycloalkoxy includes, for example, cyclopropoxy, cyclobutoxy, cyclopentoxy and cyclohexyloxy;
the tri(C1-C6 alkyl)silyl includes, for example, trimethylsilyl, triethylsilyl, tributylsilyl and *tert*-butyldimethylsilyl.

In R¹¹, R¹² and R¹³;
the halogen atom includes, for example, fluorine atom, chlorine atom and bromine atom;
the C1-C3 alkyl includes, for example, methyl, ethyl and propyl.

The phenyl substituted with R⁹, R¹⁰, R¹¹, R¹² and R¹³ includes, for example,
the phenyl wherein R⁹ and R¹⁰ are C1-C6 alkoxy, and R¹¹, R¹² and R¹³ are a hydrogen atom, halogen atom or C1-C3 alkyl;
the phenyl wherein R⁹ and R¹⁰ are independently a halogen atom, C1-C6 alkyl, C1-C6 haloalkyl, C2-C6 alkenyl, C2-C6 haloalkenyl, C2-C6 alkynyl, C2-C6 haloalkynyl, cyano C1-C6 alkyl, C1-C6 alkoxy, C1-C6 haloalkoxy, C3-C6 alkenyloxy, C3-C6 haloalkenyloxy, C3-C6 alkynyloxy, C3-C6 haloalkynyloxy, cyano C1-C6 alkoxy, C1-C6 alkylthio, C1-C6 haloalkylthio, C3-C6 alkenylthio, C3-C6 alkynylthio, C3-C6 cycloalkyl, C3-C6 cycloalkoxy, (C3-C6 cycloalkyl) C1-C3 alkoxy, C2-C6 alkoxyalkyl, tri(C1-C6 alkyl)silyl or cyano, and R¹¹, R¹² and R¹³ are hydrogen atoms;
the phenyl wherein R⁹ and R¹⁰ are independently C1-C6 alkoxy, C1-C6 haloalkoxy, C3-C6 alkenyloxy, C3-C6 haloalkenyloxy, C3-C6 alkynyloxy, C3-C6 haloalkynyloxy or cyano C1-C6 alkoxy, and R¹¹, R¹² and R¹³ are hydrogen atoms;
the phenyl wherein R⁹ and R¹⁰ are independently C1-C3 alkoxy, C3 alkenyloxy or cyano C1-C3 alkoxy, and R¹¹, R¹² and R¹³ are hydrogen atoms;
the phenyl wherein R⁹ and R¹⁰ are methoxy, and R¹¹, R¹² and R¹³ are hydrogen atoms;
the phenyl wherein R⁹ is ethoxy, R¹⁰ is methoxy, and R¹¹, R¹² and R¹³ are hydrogen atoms;
the phenyl wherein R⁹ is 2-propynyloxy, R¹⁰ is methoxy, and R¹¹, R¹² and R¹³ are hydrogen atoms;
the phenyl wherein R⁹ is cyanomethoxy, R¹⁰ is methoxy, and R¹¹, R¹² and R¹³ are hydrogen atoms.
are put up.

In R¹⁴, R¹⁵, R¹⁶ and R¹⁷;
the halogen atom includes, for example, a fluorine atom and chlorine atom;
the C1-C6 alkyl includes, for example, methyl, ethyl, propyl, isopropyl, butyl, isobutyl, *sec*-butyl, *tert*-butyl, pentyl, isopentyl and hexyl;
the C1-C6 haloalkyl includes, for example, fluoromethyl, difluoromethyl, trifluoromethyl, chlorodifluoromethyl, chlorofluoromethyl, bromodifluoromethyl, trichloromethyl, 1,1,2,2,2-pentafluoroethyl, 2,2,2-trifluoroethyl, 2,2-difluoroethyl, 2-fluoroethyl and 6,6,6-trifluorohexyl;
the C2-C6 alkenyl includes, for example, vinyl, 2-propenyl, 1-methyl-2-propenyl, 2-methyl-2-propenyl, 2-butenyl, 3-butenyl, 2-hexenyl and 5-hexenyl;
the C2-C6 haloalkenyl includes, for example, 1-chlorovinyl, 2-chlorovinyl, 2,2-dichlorovinyl, 2,2-difluorovinyl, 1,2-dichlorovinyl, 3,3-difluoro-2-propenyl and 3,3-dichloro-2-propenyl;
the C2-C6 alkynyl includes, for example, ethynyl, 3-butynyl and 5-hexynyl;
the C2-C6 haloalkynyl includes, for example, 2-chloroethynyl, 2-bromoethynyl and 6-chloro-5-hexynyl;
the C1-C6 alkoxy, for example, methoxy, ethoxy, propoxy, isopropoxy, butoxy, isobutoxy, *sec*-butoxy, *tert*-butoxy, pentyloxy, isopentyloxy and hexyloxy;
the C1-C6 haloalkoxy includes, for example, trifluoromethoxy, difluoromethoxy, bromodifluoromethoxy, chlorodifluoromethoxy, fluoromethoxy, 2,2,2-trifluoroethoxy, 1,1,2,2-tetrafluoroethoxy, 5-chloropentyloxy, 4-fluoroisopentyloxy and 2,2-dichlorohexyloxy;
the C3-C6 alkenyloxy includes, for example, 2-propenyloxy, 1-methyl-2-propenyloxy, 2-methyl-2-propenyloxy, 2-butenyloxy, 3-butenyloxy, 2-hexenyloxy and 5-hexenyloxy;
the C3-C6 haloalkenyloxy includes, for example, 3,3-difluoro-2-propenyloxy, 3,3-dichloro-2-propenyloxy, 3,3-dibromo-2-propenyloxy, 2,3-dichloro-2-propenyloxy, 6-fluoro-2-hexenyloxy and 2,2-dichloro-5-hexenyloxy;
the C3-C6 alkynyloxy includes, for example, a 2-propynyloxy, 1-methyl-2-propynyloxy, 2-butynyloxy, 3-butynyloxy, 2-hexynyloxy and 5-hexynyloxy;
the C3-C6 haloalkynyloxy includes, for example, 3-chloro-2-propynyloxy, 3-bromo-2-propynyloxy, 3-iodo-2-propynyloxy and 6-chloro-5-hexynyloxy;
the C1-C6 alkylthio includes, for example, methylthio, ethylthio, propylthio, isopropylthio, butylthio, isobutylthio, *sec*-butylthio, *tert*-butylthio, pentylthio, isopentylthio and hexylthio;
the C1-C6 haloalkylthio includes, for example, trifluoromethylthio, difluoromethylthio, bromodifluoromethylthio, chlorodifluoromethylthio, fluoromethylthio, 2,2,2-trifluoroethylthio, 1,1,2,2-tetrafluoroethylthio, 5-chloropentylthio, 4-fluoroisopentylthio and 2,2-dichlorohexylthio;
the C3-C6 cycloalkyl includes, for example, cyclopropyl, cyclobutyl, cyclopentyl and cyclohexyl;
the C3-C6 cycloalkoxy includes, for example, cyclopropoxy, cyclobutoxy, cyclopentoxy and cyclohexyloxy;
the tri(C1-C6 alkyl)silyl includes, for example, trimethylsilyl, triethylsilyl, tributylsilyl and *tert*-butyldimethylsilyl.

The *o*-phenylene substituted with R¹⁴, R¹⁵, R¹⁶ and R¹⁷ includes, for example, the *o*-phenylene wherein R¹⁴, R¹⁵, R¹⁶ and R¹⁷ are independently a hydrogen atom, halogen atom or C1-C3 alkyl;
the *o*-phenylene wherein R¹⁴ is methyl, R¹⁵ is a fluorine atom, and R¹⁶ and R¹⁷ are hydrogen atoms;
the *o*-phenylene wherein R¹⁴ is a chlorine atom, R¹⁶ is a fluorine atom, and R¹⁵ and R¹⁷ are hydrogen atoms;
the *o*-phenylene wherein R¹⁴ is methoxy, R¹⁷ is a chlorine atom, and R¹⁵ and R¹⁶ are hydrogen atoms;
the *o*-phenylene wherein R¹⁵ is a fluorine atom, R¹⁶ is a chlorine atom, and R¹⁴ and R¹⁷ are hydrogen atoms;
the *o*-phenylene wherein R¹⁶ is methoxy, R¹⁷ is methyl, and R¹⁴ and R¹⁵ are hydrogen atoms;
the *o*-phenylene wherein R¹⁴ and R¹⁵ are chlorine atoms, and R¹⁶ and R¹⁷ are hydrogen atoms;
the *o*-phenylene wherein R¹⁴ and R¹⁶ are chlorine atoms, and R¹⁵ and R¹⁷ are hydrogen atoms;
the *o*-phenylene wherein R¹⁴ and R¹⁷ are chlorine atoms, and R¹⁵ and R¹⁶ are hydrogen atoms;
the *o*-phenylene wherein R¹⁵ and R¹⁶ are chlorine atoms, and R¹⁴ and R¹⁷ are hydrogen atoms;
the *o*-phenylene wherein R¹⁶ and R¹⁷ are chlorine atoms, and R¹⁴ and R¹⁵ are hydrogen atoms;
the *o*-phenylene wherein R¹⁴ and R¹⁵ are chlorine atoms, and R¹⁶ and R¹⁷ are hydrogen atoms;
the *o*-phenylene wherein R¹⁴ and R¹⁵ are methyl, and R¹⁶ and R¹⁷ are hydrogen atoms;
the *o*-phenylene wherein R¹⁴ and R¹⁶ are methyl, and R¹⁵ and R¹⁷ are hydrogen atoms;
the *o*-phenylene wherein R¹⁴ and R¹⁷ are methyl, and R¹⁵ and R¹⁶ are hydrogen atoms;
the *o*-phenylene wherein R¹⁵ and R¹⁶ are methyl, and R¹⁴ and R¹⁷ are hydrogen atoms;
the *o*-phenylene wherein R¹⁶ and R¹⁷ are methyl, and R¹⁴ and R¹⁵ are hydrogen atoms;
the *o*-phenylene wherein R¹⁴ and R¹⁵ are methyl, and R¹⁶ and R¹⁷ are hydrogen atoms;
the *o*-phenylene wherein R¹⁴ is methyl, and R¹⁵, R¹⁶ and R¹⁷ are hydrogen atoms;
the *o*-phenylene wherein R¹⁴ is a chlorine atom, and R¹⁵, R¹⁶ and R¹⁷ are hydrogen atoms;
the *o*-phenylene wherein R¹⁴ is methoxy, and R¹⁵, R¹⁶ and R¹⁷ are hydrogen atoms;
the *o*-phenylene wherein R¹⁴ is cyano, and R¹⁵, R¹⁶ and R¹⁷ are hydrogen atoms;
the *o*-phenylene wherein R¹⁴ is trifluoromethyl, and R¹⁵, R¹⁶ and R¹⁷ are hydrogen atoms;
the *o*-phenylene wherein R¹⁵ is methyl, and R¹⁴, R¹⁶ and R¹⁷ are hydrogen atoms;
the *o*-phenylene wherein R¹⁵ is a chlorine atom, and R¹⁴, R¹⁶ and R¹⁷ are hydrogen atoms;
the *o*-phenylene wherein R¹⁵ is methoxy, and R¹⁴, R¹⁶ and R¹⁷ are hydrogen atoms;
the *o*-phenylene wherein R¹⁵ is cyano, and R¹⁴, R¹⁶ and R¹⁷ are hydrogen atoms;
the *o*-phenylene wherein R¹⁵ is trifluoromethoxy, and R¹⁴, R¹⁶ and R¹⁷ are hydrogen atoms;
the *o*-phenylene wherein R¹⁶ is methyl, and R¹⁴, R¹⁵ and R¹⁷ are hydrogen atoms;
the *o*-phenylene wherein R¹⁶ is a chlorine atom, and R¹⁴, R¹⁵ and R¹⁷ are hydrogen atoms;
the *o*-phenylene wherein R¹⁶ is methoxy, and R¹⁴, R¹⁵ and R¹⁷ are hydrogen atoms;
the *o*-phenylene wherein R¹⁶ is cyano, and R¹⁴, R¹⁵ and R¹⁷ are hydrogen atoms;
the *o*-phenylene wherein R¹⁶ is cyclopropyl, R¹⁴, R¹⁵ and R¹⁷ are hydrogen atoms;
the *o*-phenylene wherein R¹⁷ is methyl, and R¹⁴, R¹⁵ and R¹⁶ are hydrogen atoms;
the *o*-phenylene wherein R¹⁷ is a chlorine atom, and R¹⁴, R¹⁵ and R¹⁶ are hydrogen atoms;
the *o*-phenylene wherein R¹⁷ is methoxy, and R¹⁴, R¹⁵ and R¹⁶ are hydrogen atoms;
the *o*-phenylene wherein R¹⁷ is cyano, and R¹⁴, R¹⁵ and R¹⁶ are hydrogen atoms;
the *o*-phenylene wherein R¹⁷ is methylthio, and R¹⁴, R¹⁵ and R¹⁶ are hydrogen atoms;
the *o*-phenylene wherein R¹⁴, R¹⁵, R¹⁶ and R¹⁷ are hydrogen atoms.

The embodiments of the present compounds include, for example, the following compounds.

The compound of the formula (1) wherein X¹ is an oxygen atom;
the compound of the formula (1) wherein X² is an oxygen atom;
the compound of the formula (1) wherein X¹ and X² are oxygen atoms;
the compound of the formula (1) wherein R⁷ is a hydrogen atom;
the compound of the formula (1) wherein X¹ is an oxygen atom, and R⁷ is a hydrogen atom;
the compound of the formula (1) wherein X² is an oxygen atom, and R⁷ is a hydrogen atom;
the compound of the formula (1) wherein X¹ and X² are oxygen atoms, and R⁷ is a hydrogen atom;
the compound of the formula (1) wherein X¹ is an oxygen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ and X² are oxygen atoms, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein R⁷ is a hydrogen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ is an oxygen atom, R⁷ is a hydrogen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, R⁷ is a hydrogen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ and X² are oxygen atoms, R⁷ is a hydrogen atom, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ is an oxygen atom, and R⁹ is C1-C6 alkoxy;
the compound of the formula (1) wherein X² is an oxygen atom, and R⁹ is C1-C6 alkoxy;
the compound of the formula (1) wherein X¹ and X² are oxygen atoms, and R⁹ is C1-C6 alkoxy;
the compound of the formula (1) wherein R⁷ is a hydrogen atom, and R⁹ is C1-C6 alkoxy;
the compound of the formula (1) wherein X¹ is an oxygen atom, R⁷ is a hydrogen atom, and R⁹ is C1-C6 alkoxy;
the compound of the formula (1) wherein X² is an oxygen atom, R⁷ is a hydrogen atom, and R⁹ is C1-C6 alkoxy;
the compound of the formula (1) wherein X¹ and X² are oxygen atoms, R⁷ is a hydrogen atom, and R⁹ is C1-C6 alkoxy;
the compound of the formula (1) wherein X¹ is an oxygen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ and X² are oxygen atoms, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy ;
the compound of the formula (1) wherein R⁷ is a hydrogen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ is an oxygen atom, R⁷ is a hydrogen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, R⁷ is a hydrogen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ and X² are oxygen atoms, R⁷ is a hydrogen atom, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ is an oxygen atom, and R¹⁰ is C1-C6 alkoxy;
the compound of the formula (1) wherein X² is an oxygen atom, and R¹⁰ is C1-C6 alkoxy;
the compound of the formula (1) wherein X¹ and X² are oxygen atoms, and R¹⁰ is C1-C6 alkoxy;
the compound of the formula (1) wherein R⁷ is a hydrogen atom, and R¹⁰ is C1-C6 alkoxy;
the compound of the formula (1) wherein X¹ is an oxygen atom, R⁷ is a hydrogen atom, and R¹⁰ is C1-C6 alkoxy;
the compound of the formula (1) wherein X² is an oxygen atom, R⁷ is a hydrogen atom, and R¹⁰ is C1-C6 alkoxy;
the compound of the formula (1) wherein X¹ and X² are oxygen atoms, R⁷ is a hydrogen atom, and R¹⁰ is C1-C6 alkoxy;
the compound of the formula (1) wherein X¹ is an oxygen atom, and R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, and R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ and X² are oxygen atoms, and R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy ;
the compound of the formula (1) wherein R⁷ is a hydrogen atom, and R⁹ and R¹⁰ are independently C1-C6 alkoxy;
the compound of the formula (1) wherein X¹ is an oxygen atom, R⁷ is a hydrogen atom, and R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X² is an oxygen atom, R⁷ is a hydrogen atom, and R⁹ and R¹⁰ are independently C1-C6 alkoxy;
the compound of the formula (1) wherein X¹ and X² are oxygen atoms, R⁷ is a hydrogen atom, and R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein X¹ is an oxygen atom, and R⁹ and R¹⁰ are independently C1-C6 alkoxy;
the compound of the formula (1) wherein X² is an oxygen atom, and R⁹ and R¹⁰ are independently C1-C6 alkoxy;
the compound of the formula (1) wherein X¹ and X² are oxygen atoms, and R⁹ and R¹⁰ are independently C1-C6 alkoxy;
the compound of the formula (1) wherein R⁷ is a hydrogen atom, and R⁹ and R¹⁰ are independently C1-C6 alkoxy;
the compound of the formula (1) wherein X¹ is an oxygen atom, R⁷ is a hydrogen atom, and R⁹ and R¹⁰ are independently C1-C6 alkoxy;
the compound of the formula (1) wherein X² is an oxygen atom, R⁷ is a hydrogen atom, and R⁹ and R¹⁰ are independently C1-C6 alkoxy;
the compound of the formula (1) wherein X¹ and X² are oxygen atoms, R⁷ is a hydrogen atom, and R⁹ and R¹⁰ are independently C1-C6 alkoxy;
the compound of the formula (1) wherein R⁴ and R⁵ are hydrogen atoms;
the compound of the formula (1) wherein R⁴ and R⁵ are hydrogen atoms, and X¹ is an oxygen atom;
the compound of the formula (1) wherein R⁴ and R⁵ are hydrogen atoms, and X² is an oxygen atom;
the compound of the formula (1) wherein R⁴ and R⁵ are hydrogen atoms, and X¹ and X 2 are oxygen atoms;
the compound of the formula (1) wherein R⁴, R⁵ and R⁷ are hydrogen atoms, and X¹ is an oxygen atom;
the compound of the formula (1) wherein R⁴, R⁵ and R⁷ are hydrogen atoms, and X² is an oxygen atom;
the compound of the formula (1) wherein R⁴, R⁵ and R⁷ are hydrogen atoms, and X¹ and X² are oxygen atoms;
the compound of the formula (1) wherein R⁴ and R⁵ are hydrogen atoms, and R⁹ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein R⁴ and R⁵ are hydrogen atoms, and R¹⁰ is C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein R⁴ and R⁵ are hydrogen atoms, and R⁹ and R¹⁰ are C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy;
the compound of the formula (1) wherein R⁴ and R⁵ are hydrogen atoms, and R⁹ is C1-C6 alkoxy;
the compound of the formula (1) wherein R⁴ and R⁵ are hydrogen atoms, and R¹⁰ is C1-C6 alkoxy;
the compound of the formula (1) wherein R⁴ and R⁵ are hydrogen atoms, and R⁹and R¹⁰ are C1-C6 alkoxy;
the compound of the formula (1) wherein X¹ and X² are oxygen atoms, R⁷ is a hydrogen atom, R⁴ and R⁵ are hydrogen atoms, and R⁹ is C1-C6 alkoxy;
the compound of the formula (1) wherein X¹ and X² are oxygen atoms, R⁷ is a hydrogen atom, R⁴ and R⁵ are hydrogen atoms, and R¹⁰ is C1-C6 alkoxy;
the compound of the formula (1) wherein X¹ and X² are oxygen atoms, R⁷ is a hydrogen atom, R⁴ and R⁵ are hydrogen atoms, and R⁹ and R¹⁰ are C1-C6 alkoxy;
the compound of the formula (1) wherein R¹¹, R¹² and R¹³ are hydrogen atoms;
the compound of the formula (1) wherein R⁴, R⁵, R¹¹, R¹² and R¹³ are hydrogen atoms;
the compound of the formula (1) wherein R⁷, R¹¹, R¹² and R¹³ are hydrogen atomss;
the compound of the formula (1) wherein R⁴, R⁵, R⁷, R¹¹, R¹² and R¹³ are hydrogen atoms;
the compound of the formula (1) wherein X¹ and X² is oxygen atoms, and R⁴, R⁵, R⁷, R¹¹, R¹² and R¹³ are hydrogen atoms;
the compound of the formula (1) wherein R¹⁴, R¹⁵, R¹⁶ and R¹⁷ are independently a hydrogen atom, halogen atom or C1-C6 alkyl;
the compound of the formula (1) wherein R¹¹, R¹² and R¹³ are hydrogen atoms, and R¹⁴, R¹⁵, R¹⁶ and R¹⁷ are independently a hydrogen atom, halogen atom or C1-C6 alkyl;
the compound of the formula (1) wherein R⁴, R⁵, R¹¹, R¹² and R¹³ is hydrogen atoms, and R¹⁴, R¹⁵, R¹⁶ and R¹⁷ are independently a hydrogen atom, halogen atom or C1-C6 alkyl;
the compound of the formula (1) wherein R⁴, R⁵ and R⁷ is hydrogen atoms, and R¹⁴, R¹⁵, R¹⁶ and R¹⁷ are independently a hydrogen atom, halogen atom or C1-C6 alkyl;
the compound of the formula (1) wherein R⁴, R⁵, R⁷, R¹¹, R¹² and R¹³ is hydrogen atoms, and R¹⁴, R¹⁵, R¹⁶ and R¹⁷ is independently a hydrogen atom, halogen atom or C1-C6 alkyl;
the compound of the formula (1) wherein R¹⁴, R¹⁵, R¹⁶ and R¹⁷ are hydrogen atoms;
the compound of the formula (1) wherein R⁴, R⁵, R¹⁴, R¹⁵, R¹⁶ and R¹⁷ are hydrogen atoms;
the compound of the formula (1) wherein R¹¹, R¹², R¹³, R¹⁴, R¹⁵, R¹⁶ and R¹⁷ are hydrogen atoms;
the compound of the formula (1) wherein R⁷, R¹⁴, R¹⁵, R¹⁶ and R¹⁷ are hydrogen atoms;
the compound of the formula (1) wherein R⁴, R⁵, R⁷, R¹⁴, R¹⁵, R¹⁶ and R¹⁷ are hydrogen atoms;
the compound of the formula (1) wherein R⁴, R⁵, R¹¹, R¹², R¹³, R¹⁴, R¹⁵, R¹⁶ and R¹⁷ are hydrogen atoms;
the compound of the formula (1) wherein R⁴, R⁵, R⁷, R¹⁴, R¹⁵, R¹⁶ and R¹⁷ are hydrogen atoms;
the compound of the formula (1) wherein R¹⁴, R¹⁵, R¹⁶ and R¹⁷ are independently a hydrogen atom, halogen atom or a C1-C6 alkyl;
the compound of the formula (1) wherein R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy, R¹¹, R¹² and R¹³ are hydrogen atoms, and R¹⁴, R¹⁵, R¹⁶ and R¹⁷ are independently a hydrogen atom, halogen atom or C1-C6 alkyl;
the compound of the formula (1) wherein R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy, R⁴, R⁵, R¹¹, R¹² and R¹³ are hydrogen atoms, and R¹⁴, R¹⁵, R¹⁶ and R¹⁷ are independently a hydrogen atom, halogen atom or C1-C6 alkyl;
the compound of the formula (1) wherein R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy, R⁴, R⁵ and R⁷ are hydrogen atoms, and R¹⁴, R¹⁵, R¹⁶ and R¹⁷ are independently a hydrogen atom, halogen atom or C1-C6 alkyl;
the compound of the formula (1) wherein R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy, R⁴, R⁵, R⁷, R¹¹, R¹² and R¹³ are hydrogen atoms, and R¹⁴, R¹⁵, R¹⁶ and R¹⁷ are independently a hydrogen atom, halogen atom or C1-C6 alkyl;
the compound of the formula (1) wherein X¹ and X² are oxygen atoms, R⁹ and R¹⁰ are independently C1-C6 alkoxy, C1-C6 haloalkoxy, C2-C6 alkenyloxy, C2-C6 haloalkenyloxy, C2-C6 alkynyloxy, C2-C6 haloalkynyloxy or cyano C1-C6 alkoxy, R⁴, R⁵, R⁷, R¹¹, R¹² and R¹³ are hydrogen atoms, and R¹⁴, R¹⁵, R¹⁶ and R¹⁷ are independently a hydrogen atom, halogen atom or C1-C6 alkyl.

The following describes a production process for the present compound.

The present compound can be produced by, for example, (Production Process A), (Production Process B), (Production Process C) or (Production Process D).

### (Production Process A)

The present compound of the formula (4) can be produced by making the compound of the formula (2) react with the compound of the formula (3). 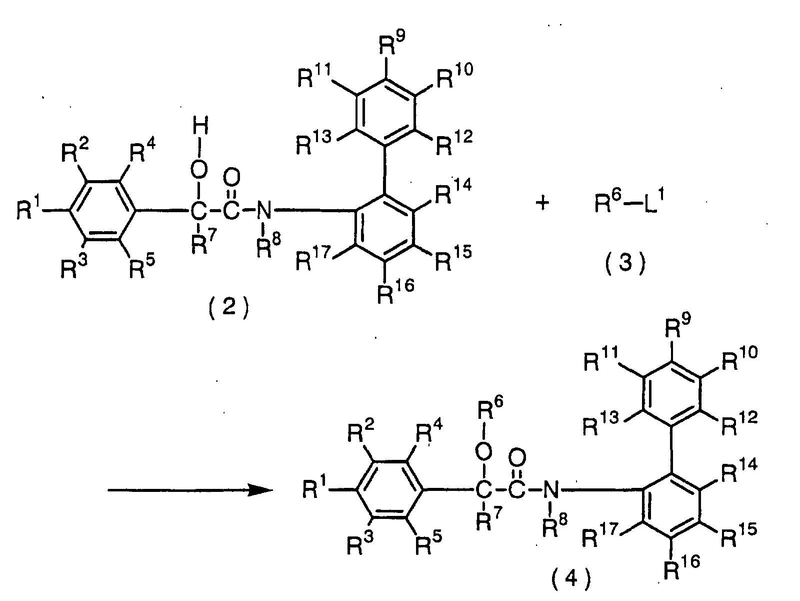 wherein L¹ represents a chlorine atom, bromine atom or methanesulfonyloxy, and R¹ to R¹⁷ have the same meaning as described above.

The reaction is carried out in the presence or absence of a solvent, and usually in the presence of base.

The solvent used in the reaction includes, for example, ethers such as 1,4-dioxane, tetrahydrofuran, ethylene glycol dimethyl ether, *tert*-butyl methyl ether and the like; aliphatic hydrocarbons such as hexane, heptane, octane and the like; aromatic hydrocarbons such as toluene, xylene and the like; halogenated hydrocarbons such as chlorobenzene and the like; esters such as ethyl acetate, butyl acetate and the like; nitriles such as acetonitrile, butyronitrile and the like; acid amides such as N,N-dimethylformamide and the like; sulfoxides such as dimethylsulfoxide and the like; and the mixture thereof.

The base used in the reaction includes, for example, carbonates such as sodium carbonate, potassium carbonate and the like; alkali metal hydrides such as sodium hydride and the like; tertiary amines such as triethylamine, diisopropylethamine, 1,8-diazabicyclo[5.4.0]undec-7-ene, 1,5-diazabicyclo[4.3.0]non-5-ene and the like; nitrogen containing aromatic compounds such as pyridine, 4-dimethylaminopyridine and the like.

Concerning the ratio of the compound used in the reaction, the amount of the compound of the formula (3) is usually 1 to 10 mole, and the amount of the base is usually 1 to 10 mole, based on 1 mole of the compound of the formula (2).

The reaction time is usually in the range of 0.1 to 24 hours, and the reaction temperature is usually in the range of 0 to 150°C.

After completion of the reaction, the reaction mixture is subjected to usual post treatment procedure such as extracting with organic solvent, subsequently drying and concentrating the organic layer, to isolate the present compound of the formula (4). Even more, the isolated present compound of the formula (4) can be purified by a technique such as chromatography, recrystallization and the like.

### (Production Process B)

The present compound of the formula (6) can be produced by making the compound of the formula (5) react with
2,4-bis(4-methoxyphenyl)-1,3-dithia-2,4-diphosphetane-2,4-disulfide (Lawesson's reagent; hereinafter referred to as Lawesson's reagent). 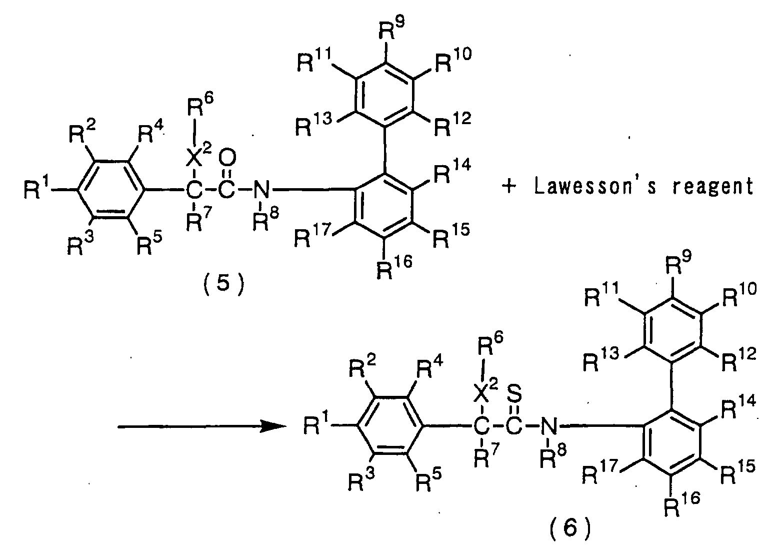 wherein R¹ to R¹⁷ and X² have the same meaning as described above.

The reaction is usually carried out in solvents. The solvent used in the reaction includes, for example, ethers such as 1,4-dioxane, tetrahydrofuran, ethylene glycol dimethyl ether, *tert*-butyl methyl ether and the like; aliphatic hydrocarbons such as hexane, heptane, octane and the like; aromatic hydrocarbons such as toluene, xylene and the like; halogenated hydrocarbons such as chlorobenzene and the like; nitriles such as acetonitrile, butyronitrile and the like; sulfoxides such as dimethylsulfoxide and the like; and the mixture thereof.

The amount of Lawesson's reagent is usually 1 to 10 mole based on 1 mole of the compound of the formula (5).

The reaction time is usually in the range of 0.5 to 24 hours, and the reaction temperature is usually in the range of 50 to 150 °C.

After completion of the reaction, the reaction mixture is subjected to usual post treatment procedure such as extracting with organic solvent, subsequently drying and concentrating the organic layer, to isolate the present compound of the formula (6). Even more, the isolated present compound of the formula (6) can be purified by a technique such as chromatography, recrystallization and the like.

### (Production Process C)

The present compound of the formula (9) can be produced by making the compound of the formula (7) react with the compound of the formula (8). 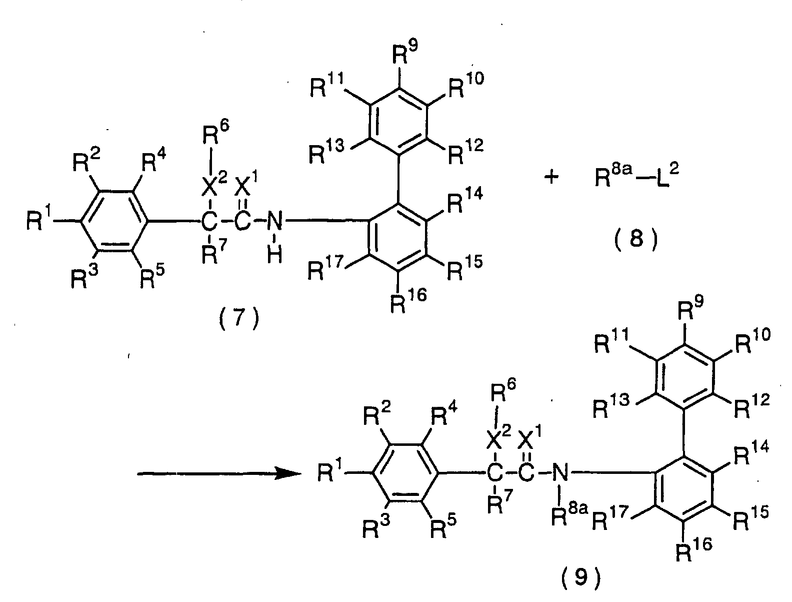 wherein L² represents a bromine atom, iodine atom or methanesulfonyloxy, R^{8a} represents C1-C3 alkyl, R¹ to R⁷, R⁹ to R¹⁷, X¹ and X² have the same meaning as described above.

The reaction is carried out in the presence or absence of a solvent, and usually in the presence of base.

The solvent used in the reaction includes, for example, ethers such as 1,4-dioxane, tetrahydrofuran, ethylene glycol dimethyl ether, tert-butyl methyl ether and the like; aliphatic hydrocarbons such as hexane, heptane, octane and the like; aromatic hydrocarbons such as toluene, xylene and the like; halogenated hydrocarbons such as chlorobenzene and the like; esters such as ethyl acetate, butyl acetate and the like; nitriles such as acetonitrile, butyronitrile and the like; acid amides such as N,N-dimethylformamide and the like; sulfoxides such as dimethylsulfoxide and the like; and the mixture thereof.

The base used in the reaction includes, for example, carbonates such as sodium carbonate, potassium carbonate and the like; alkali metal hydrides such as sodium hydride and the like.

Concerning the ratio of the compound used in the reaction, the amount of the compound of the formula (8) is usually 1 to 10 mole, and the amount of the base is usually 1 to 10 mole, based on 1 mole of the compound of the formula (7).

The reaction time is usually in the range of 0.5 to 24 hours, and the reaction temperature is usually in the range of 0 to 120°C.

After completion of the reaction, the reaction mixture is subjected to usual post treatment procedure such as extracting with organic solvent, subsequently drying and concentrating the organic layer, to isolate the present compound of the formula (9). Even more, the isolated present compound of the formula (9) can be purified by a technique such as chromatography, recrystallization and the like.

### (Production Process D)

The present compound of the formula (13) can be produced by making the compound of the formula (10) react with methanesulfonyl chloride, and subsequently making react with the compound of the formula (12). 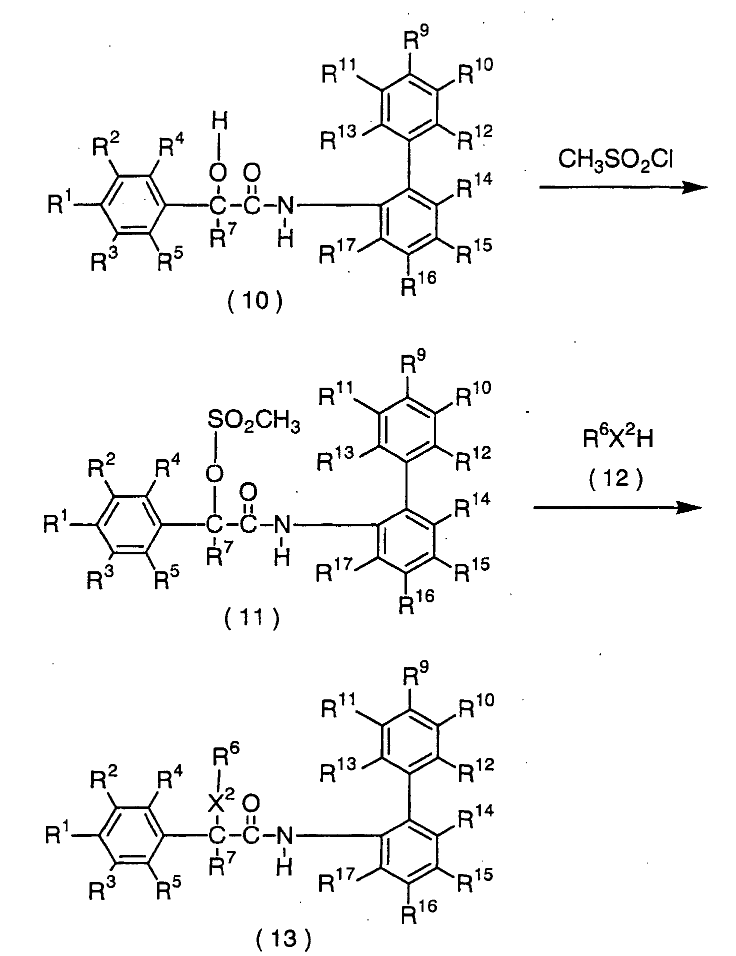 wherein R¹ to R⁷, R⁹ to R¹² and X² have the same meaning as described above.

### [Step 1]: The process of making the compound of the formula (10) react with methanesulfonyl chloride.

The reaction is carried out usually in a solvent, and usually in the presence of base.

The solvent used in the reaction includes, for example, ethers such as 1,4-dioxane, tetrahydrofuran, ethylene glycol dimethyl ether, tert-butyl methyl ether and the like; aliphatic hydrocarbons such as hexane, heptane, octane and the like; aromatic hydrocarbons such as toluene, xylene and the like; halogenated hydrocarbons such as chlorobenzene and the like; esters such as ethyl acetate, butyl acetate and the like; nitriles such as acetonitrile, butyronitrile and the like; acid amides such as N,N-dimethylformamide and the like; sulfoxides such as dimethylsulfoxide and the like; and the mixture thereof.

The base used in the reaction includes, for example, carbonates such as sodium carbonate, potassium carbonate and the like; alkali metal hydrides such as sodium hydride and the like; tertiary amines such as triethylamine, diisopropylethamine, 1,8-diazabicyclo[5.4.0]undec-7-ene, 1,5-diazabicyclo[4.3.0]non-5-ene and the like; nitrogen containing aromatic compounds such as pyridine, 4-dimethylaminopyridine and the like.

Concerning the ratio of the compound used in the reaction, the amount of the base is usually 1 to 10 mole, and the amount of methanesulfonyl chloride is usually 1 to 3 mole, based on 1 mole of the compound of the formula (10).

The reaction time is usually in the range of 1 to 24 hours, and the reaction temperature is usually in the range of -20 to 100°C.

After completion of the reaction, the reaction mixture is subjected to usual post treatment procedure such as extracting with organic solvent, subsequently drying and concentrating the organic layer, to isolate the present compound of the formula (11). Even more, the isolated present compound of the formula (11) can be purified by a technique such as chromatography, recrystallization and the like.

### [Step 2]: The process of making the compound of the formula (11) react with the compound of the formula (12).

The reaction is carried out in the presence or absence of a solvent, and in the presence or absence of base.

The solvent used in the reaction, if required, includes, for example, ethers such as 1,4-dioxane, tetrahydrofuran, ethylene glycol dimethyl ether, tert-butyl methyl ether and the like; aliphatic hydrocarbons such as hexane, heptane, octane and the like; aromatic hydrocarbons such as toluene, xylene and the like; halogenated hydrocarbons such as chlorobenzene and the like; esters such as ethyl acetate, butyl acetate and the like; nitriles such as acetonitrile, butyronitrile and the like; acid amides such as N,N-dimethylformamide and the like; sulfoxides such as dimethylsulfoxide and the like; and the mixture thereof.

The base used in the reaction, if required, includes, for example, carbonates such as sodium carbonate, potassium carbonate and the like; tertiary amines such as triethylamine, diisopropylethamine, 1,8-diazabicyclo[5.4.0]undec-7-ene, 1,5-diazabicyclo[4.3.0]non-5-ene and the like; nitrogen containing aromatic compounds such as pyridine, 4-dimethylaminopyridine and the like.

The amount of the compound of the formula (12) is usually 1 to 10 mole based on 1 mole of the compound of the formula (11).

The reaction time is usually in the range of 1 to 24 hours, and the reaction temperature is usually in the range of 0 to 150°C.

After completion of the reaction, the reaction mixture is subjected to usual post treatment procedure such as extracting with organic solvent, subsequently drying and concentrating the organic layer, to isolate the present compound of the formula (13). Even more, the isolated present compound of the formula (13) can be purified by a technique such as chromatography, recrystallization and the like.

The following describes a production process for the intermediate compound of the present compound.

The compound of the formula (19) can be produced by, for example, the following scheme. 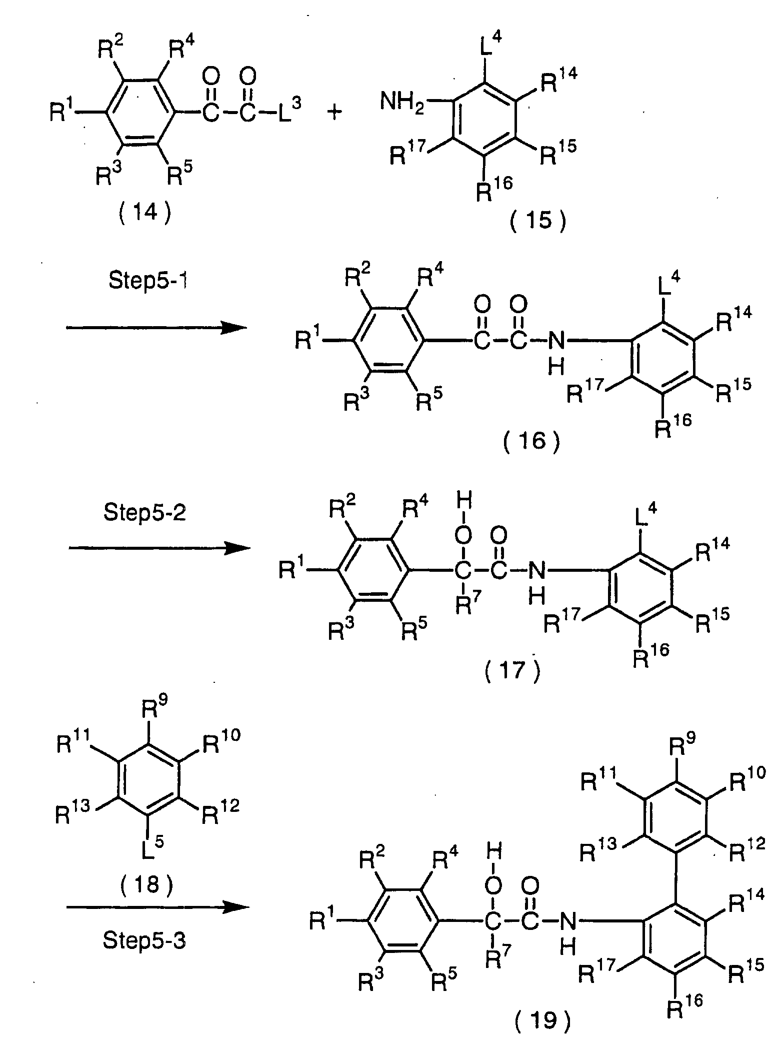 wherein L³ represents a chlorine atom or bromine atom, L⁴ represents a chlorine atom, bromine atom, iodine atom or trifluoromethanesulfonyloxy, L⁵ represents B(OH)₂, B(OR¹⁹)₂ or SnR²⁰, R¹⁹ and R²⁰ represent C1-C10 alkyl, and R¹ to R⁵, R⁷ and R⁹ to R¹⁷ have the same meaning as the described above.

### [Step5-1]

The compound of the formula (16) can be produced by making the compound of the formula (14) react with the compound of the formula (15).

The reaction is carried out usually in solvents, and usually in the presence of base.

The solvent used in the reaction includes, for example, ethers such as 1,4-dioxane, tetrahydrofuran, ethylene glycol dimethyl ether, tert-butyl methyl ether and the like; aliphatic hydrocarbons such as hexane, heptane, octane and the like; aromatic hydrocarbons such as toluene, xylene and the like; halogenated hydrocarbons such as chlorobenzene and the like; esters such as ethyl acetate, butyl acetate and the like; nitriles such as acetonitrile, butyronitrile and the like; acid amides such as N,N-dimethylformamide; sulfoxides such as dimethylsulfoxide and the like; and the mixture thereof.

The base used in the reaction includes, for example, carbonates such as sodium carbonate, potassium carbonate and the like; tertiary amines such as triethylamine, diisopropylethamine, 1,8-diazabicyclo[5.4.0]undec-7-ene, 1,5-diazabicyclo[4.3.0]non-5-ene and the like; nitrogen containing aromatic compounds such as pyridine, 4-dimethylaminopyridine and the like.

Concerning the ratio of the compound used in the reaction, the amount of the base is usually 1 to 10 mole, and the amount of the compound of the formula (15) is usually 1 to 3 mole, based on 1 mole of the compound of the formula (14).

The reaction time is usually in the range of 1 to 24 hours, and the reaction temperature is usually in the range of -20 to 100 °C.

After completion of the reaction, the reaction mixture is subjected to usual post treatment procedure such as extracting with organic solvent, subsequently drying and concentrating the organic layer, to isolate the present compound of the formula (16). Even more, the isolated present compound of the formula (16) can be purified by a technique such as chromatography, recrystallization and the like.

### [Step5-2]

The compound of the formula (17) wherein R⁷ is a hydrogen atom can be produced by making the compound of the formula (16) react with a reducing agent.

The reaction is carried out usually in solvents.

The solvent used in the reaction includes, for example, ethers such as 1,4-dioxane, tetrahydrofuran, ethylene glycol dimethyl ether, *tert*-butyl methyl ether and the like; aliphatic hydrocarbons such as hexane, heptane, octane and the like; aromatic hydrocarbons such as toluene, xylene and the like; water; and the mixture thereof.

The reducing agent used in the reaction includes, for example, sodium borohydride and potassium borohydride.

The amount of the reducing agent is usually 0.25 to 3 mole based on 1 mole of the compound of the formula (16).

The reaction time is usually in the range of instant to 24 hours, and the reaction temperature is usually in the range of-20 to 100 °C.

After completion of the reaction, the reaction mixture is subjected to usual post treatment procedure such as extracting with organic solvent, subsequently drying and concentrating the organic layer, to isolate the present compound of the formula (17). Even more, the isolated present compound of the formula (17) can be purified by a technique such as chromatography, recrystallization and the like.

The compound of the formula (17) wherein R⁷ is C1-C3 alkyl can be produced by making the compound of the formula (16) react with an organic metal compound such as Grignard reagent, alkyl lithium and the like.

The reaction is carried out usually in solvents.

The solvent used in the reaction includes, for example, ethers such as 1,4-dioxane, tetrahydrofuran, ethylene glycol dimethyl ether, *tert-*butyl methyl ether and the like; aliphatic hydrocarbons such as hexane, heptane, octane and the like; aromatic hydrocarbons such as toluene, xylene and the like.

The amount of the organic metal compound is usually 1 to 3 mole based on 1 mole of the compound of the formula (16).

The reaction time is usually in the range of instant to 24 hours, and the reaction temperature is usually in the range of -80 to 50 °C.

After completion of the reaction, the reaction mixture is subjected to usual post treatment procedure such as extracting with organic solvent, subsequently drying and concentrating the organic layer, to isolate the present compound of the formula (17). Even more, the isolated present compound of the formula (17) can be purified by a technique such as chromatography, recrystallization and the like.

### [Step5-3]

The compound of the formula (19) can be produced by making the compound of the formula (17) react with the compound of the formula (18) in the presence of a catalyst.

The reaction is carried out usually in a solvent, and usually in the presence of base.

The solvent used in the reaction includes, for example, ethers such as 1,4-dioxane, tetrahydrofuran, ethylene glycol dimethyl ether, *tert*-butyl methyl ether and the like; aliphatic hydrocarbons such as hexane, heptane, octane and the like; aromatic hydrocarbons such as toluene, xylene and the like; halogenated hydrocarbons such as chlorobenzene and the like; nitriles such as acetonitrile, butyronitrile and the like; acid amides such as N,N-dimethylformamide; sulfoxides such as dimethylsulfoxide and the like; and the mixture thereof.

The catalyst used in the reaction includes, for example, palladium compounds such as palladium acetate, tetrakis(triphenylphosphine) palladium, {1,1-bis(diphenylphosphino)ferrocene}palladium dichloride methylene chloride complex, bis(triphenylphosphine)palladium dichloride and the like.

Concerning the ratio of the compound used in the reaction, the amount of the compound of the formula (17) is usually 1 to 5 mole, and the amount of the catalyst is usually 0.001 to 0.1 mole, based on 1 mole of the compound of the formula (18).

As the need arises, the reaction may be carried out in the presence of a base (for example, an inorganic base such as sodium acetate, potassium acetate, potassium phosphate, sodium hydrogencarbonate and the like) and/or phase transfer catalyst (for example, tertiary ammonium salt such as tetrafluorobenzyl bromide, benzyl triethylammonium bromide and the like).

The reaction time is usually in the range of 0.2 to 24 hours, and the reaction temperature is usually in the range of 50 to 120 °C.

After completion of the reaction, the reaction mixture is subjected to usual post treatment procedure such as extracting with organic solvent, subsequently drying and concentrating the organic layer, to isolate the present compound of the formula (19). Even more, the isolated present compound of the formula (19) can be purified by a technique such as chromatography, recrystallization and the like.

The compound of the formula (14) can be produced by, for example, the following scheme. 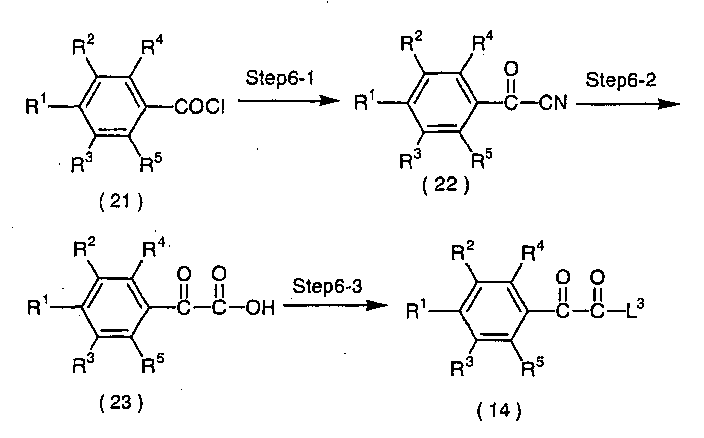 wherein R¹ to R⁵ and L³ have the same meaning as the described above.

### [Step 6-1]

The compound of the formula (22) can be produced by making the compound of the formula (21) react with a cyanide compound.

The reaction is carried out usually in solvents.

The solvent used in the reaction includes, for example, ethers such as 1,4-dioxane, tetrahydrofuran, ethylene glycol dimethyl ether, tert-butyl methyl ether and the like; aliphatic hydrocarbons such as hexane, heptane, octane and the like; aromatic hydrocarbons such as toluene, xylene and the like; nitriles such as acetonitrile, butyronitrile and the like; acid amides such as N,N-dimethylformamide; sulfoxides such as dimethylsulfoxide and the like; and the mixture thereof.

The cyanide compound used in the reaction includes, for example, alkali metal cyanide such as sodium cyanide, potassium cyanide and the like; and cupric cyanide.

The amount of the cyanide compound is usually 1 to 5 mole based on 1 mole of the compound of the formula (21).

The reaction time is usually in the range of 1 to 24 hours, and the reaction temperature is usually in the range of 50 to 100 °C.

After completion of the reaction, the reaction mixture is subjected to usual post treatment procedure such as extracting with organic solvent, subsequently drying and concentrating the organic layer, to isolate the present compound of the formula (22). Even more, the isolated present compound of the formula (22) can be purified by a technique such as chromatography, recrystallization and the like.

### [Step 6-2]

The compound of the formula (23) can be produced by making the compound of the formula (22) hydrolyze under acidic condition.

The reaction is carried out usually in the presence of an acid and water, and, if necessary, in a organic solvents.

The acid used in the reaction includes, for example, hydrochloric acid, hydrobromic acid and sulfuric acid.

The amount of the acid is usually 1 mole to excess amount based on 1 mole of the compound of the formula (22).

The reaction time is usually in the range of 1 to 40 hours, and the reaction temperature is usually in the range of 40 to 100 °C.

After completion of the reaction, the reaction mixture is subjected to usual post treatment procedure such as extracting with organic solvent, subsequently drying and concentrating the organic layer, to isolate the present compound of the formula (23). Even more, the isolated present compound of the formula (23) can be purified by a technique such as chromatography, recrystallization and the like.

### [Step 6-3]

The compound of the formula (14) can be produced by making the compound of the formula (23) react with a chlorinating agent or brominating agent.

The reaction is carried out usually in the presence or absence of a solvent.

The solvent used in the reaction includes, for example, ethers such as 1,4-dioxane, tetrahydrofuran, ethylene glycol dimethyl ether, *tert*-butyl methyl ether and the like; aliphatic hydrocarbons such as hexane, heptane, octane and the like; aromatic hydrocarbons such as toluene, xylene and the like; halogenated hydrocarbons such as chlorobenzene and the like; nitriles such as acetonitrile, butyronitrile and the like; and the mixture thereof.

The chlorinating agent and brominating agent used in the reaction includes, for example, thionyl chloride, phosphorous oxychloride, phosphorous chloride and thionyl bromide.

The amount of the chlorinating agent or brominating agent used in the reaction is usually 1 to 5 mole based on 1 mole of the compound of the formula (23).

The reaction time is usually in the range of 0.1 to 24 hours, and the reaction temperature is usually in the range of 50 to 150 °C.

After completion of the reaction, the reaction mixture is subjected to usual post treatment procedure such as extracting with organic solvent, subsequently drying and concentrating the organic layer, to isolate the present compound of the formula (14). Even more, the isolated present compound of the formula (14) can be purified by a technique such as chromatography, recrystallization and the like.

Examples of the plant diseases to be controlled by the present compound include, for example,
*Pyricularia oryzae* and *Cochliobolus miyabeanus* and *Rhizoctonia solani* of rice; *Erysiphe graminis, Gibberella zeae, Puccinia striiformis, P. graminis, P. recondita, P. hordei, Typhula sp., Micronectriella nivalis, Ustilago tritici, U. nuda, Tilletia caries, Pseudocercosporella herpotrichoides, Rhynchosporium secalis, Septoria tritici* and *Leptosphaeria nodorum*, of wheat and barley; *Diaporthe citri, Elsinoe fawcetti, Penicillium digitatum* and *P. italicum* of citrus; *Sclerotinia mali, Valsa mali, Podosphaera leucotricha, Alternaria mali* and *Venturia inaequalis* of apple; *Venturia nashicola, V. pirina, Alternaria kikuchiana* and *Gymnosporangium haraeanum* of pear; *Sclerotinia cinerea, Cladosporium carpophilum* and *Phomopsis sp.* of peach; *Elsinoe ampelina, Glomerella cingulata,* *Uncinula necator, Phakopsora ampelopsidis, Guignardia bidwellii* and *Plasmopara viticola*, of grape; *Gloeosporium kaki, Cercospora kaki* and *Mycosphaerella nawae* of Japanese persimmon; *Colletotrichum lagenarium, Sphaerothecafuliginea, Mycosphaerella melonis, Fusarium oxysporum, Pseudoperonospora cubensis, Phytophthora sp. and Pythium sp*. of gourd; *Alternaria solani, Cladosporium fulvum* and *Phytophthora infestans* of tomato; *Phomopsis vexans* and *Erysiphe cichoracearum*, of eggplant; *Alternaria japonica* and *Cercosporella brassicae* of Cruciferae vegetables; *Puccinia allii* of leek; *Cercospora kikuchii, Elsinoe glycines* and *Diaporthe phaseolorum var. sojae* of soybean; *Colletotrichum lindemthianum* of kidney bean; *Cercospora personata* and *Cercospora arachidicola* of peanut; *Erysiphe pisi* of pea; *Alternaria solani* and *Phytophthora infestans* of potato; *Sphaerotheca humuli* of strawberry; *Exobasidium reticulatum* and *Elsinoe leucospila* of tea; *Alternaria longipes, Erysiphe cichoracearum, Colletotrichum tabacum, Peronospora tabacina* and *Phytophthora nicotianae* of tobacco; *Cercospora beticola* of sugar beet; *Diplocarpon rosae* and *Sphaerotheca pannosa* of rose; *Septoria chrysanthemi-indici* and *Puccinia horiana* of chrysanthemum; and *Botrytis cinerea* and *Sclerotinia sclerotiorum* of various crops.

When the present compound is used for controlling plant diseases by applying the present compound, it can be used as the present compound itself, but usually as a fungicidal composition containing the present compound and an appropriate carrier. The fungicidal composition of the present invention is usually formulated to emulsifiable concentrates, wettable powders, water dispersible granules, flowables, dusts, granules and so on by mixing with solid carrier, liquid carrier, surfactant or the other auxiliaries. These formulations usually contain 0.1 to 90% by weight of the present compound.

Examples of the solid carrier utilized for the formulation include fine powders or granules of minerals such as kaolin clay, attapulgite clay, bentonite, montmorillonite, terra alba, pyrophyllite, talc, diatomaceous earth and calcite; natural organic substances such as corncob and walnut shell; synthetic organic substances such as urea; salts such as calcium carbonate and ammonium sulfate; and synthetic inorganic substances such as synthetic hydrous silicon oxide. Examples of the liquid carrier include aromatic hydrocarbons such as xylene, alkylbenzene and methylnaphthalene; alcohols such as isopropanol, ethylene glycol, propylene glycol and cellosolve; ketones such as acetone, cyclohexanone and isophorone; vegetable oils such as soybean oil and cottonseed oil; paraffin type aliphatic hydrocarbons; esters; dimethyl sulfoxide; acetonitrile and water.

Examples of the surfactant include anionic surfactants such as alkylsulfate ester salts, alkylarylsulfonate salts, dialkyl sulfosaccinate salts, polyoxyethylenealkylary ether phosphate salts, ligninsulfonate salts and naphthalenesulfonate formaldehyde condensate; nonionic surfactants such as polyoxyethylenealkylary ether, polyoxyethylenealkylpolyoxypropylene block copolymers and sorbitan fatty acid esters.

Examples of the auxiliaries for formulation include water soluble polymers such as polyvinyl alcohol and polyvinylpyrrolidone; polysaccharides such as gum arabic, alginic acid and its salts, CMC (carboxymethylcellulose) and xanthan gum; inorganic substances such as aluminium magnesium silicate and alumina sol; preservatives; coloring agent; PAP (isopropyl acid phosphate) and stabilizers such as BHT.

By applying the fungicidal composition of the present invention to a plant, the plant can be protected from plant diseases, namely the plant diseases can be controlled. By applying the fungicidal composition of the present invention to a soil, the plant growing on the soil can be protected from plant diseases, namely the plant diseases can be controlled.

When the plant disease controlling composition of the present invention is used with foliar application to a plant or soil application, the application amount, although it may vary with a kind of crops, a kind of plant disease to be controlled, an extent of breaking out plant disease to be controlled, formulation types, the time of application, the weather conditions and the like, is usually in the range of 1 to 5,000 g, preferably 5 to 1,000 g, 10,000 m².

The emulsifiable concentrates, wettable powders, flowables and the like are usually applied by spraying after diluted with water. In this case, the concentrate of the present compound is usually in the range of 0.0001 to 3 weight%, preferably 0.0005 to 1 weight%. The dusts, granules and the like are usually applied as such without any dilution.

The present fungicidal composition of the present invention can be used with the other application such as seed treatment. The method includes, for example, soaking seeds in the fungicidal composition of the present invention of 1 to 1000ppm , spraying or daubing the fungicidal composition of the present invention of 1 to 1000ppm on seeds, and powder coating seeds with the fungicidal composition of the present invention.

The method for controlling plant diseases of the present invention is usually carried out by applying an effective amount of the present compound on the plant wherein occurrence of a plant disease is predictable, and/or the soil wherein the plant is growing.

The fungicidal composition of the present invention can be used as agricultural/horticultural plant diseases controlling composition, namely plant diseases controlling composition in the plowed fields, paddy fields, orchards, tea plantations, pastures, lawns and the like.

Also, the fungicidal composition of the present invention can be used with other plant diseases controlling composition, insecticides, acaricides, nematocides, herbicides, plant growth regulators and/or fertilizers.

Examples of such other plant diseases controlling composition include: chlorothalonil; fluazinam; dichlofluanid; fosetyl-Al; cyclic imide derivatives such as captan, captafol, folpet and the like; dithiocarbamate derivatives such as maneb, mancozeb, thiuram, ziram, zineb, propineb and the like; inorganic or organic copper derivatives such as basic copper sulfate, basic copper chloride, copper hydroxide, copper-oxinate and the like; acylalanine derivatives such as metalaxyl, furalaxyl, ofurace, cyprofuram, benalaxyl, oxadixyl and the like; strobilurine compound such as kresoxim-methyl, azoxystrobin, trifloxystrobin, picoxystrobin, pyraclostrobin, dimoxystrobin and the like; anilinopyrimidine derivatives such as cyprodinil, pyrimethanil, mepanipyrim and the like; phenyl pyrrole derivatives such as fenpiclonil, fludioxonil and the like; imide derivatives such as procymidone, iprodione, vinclozolin and the like; benzimidazole derivatives such as carbendazim, benomyl, thiabendazole, thiophanate methyl and the like; amine derivatives such as fenpropimorph, tridemorph, fenpropidin, spiroxamine and the like; azole derivatives such as propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol and the like; cymoxanil; dimethomorph; famoxyadone, fenamidone; iprovalicarb; benthiavalicarb; cyazofamid, zoxamide, ethaboxam; nicobifen; fenhexamid; quinoxyfen; diethofencarb and acibenzolar S-methyl.

The following production examples, formulation examples and test examples and the like describe the invention further in detail below, but do not limit the scope of the invention.

At first the production examples of the present compound is shown. In addition, a number of the present compound is a compound number as described in the following tables.

### Production Example 1

In 10 ml of diethyl ether were added 3.64 g of N-3',4'-dimethoxybiphenyl-2-yl)-2-hydroxy-2-(4-methylphenyl)acetamide and 0.30 ml of triethylamine, and 0.15 ml of methanesulfonyl chloride was added dropwise at about 0°C. The mixture was stirred at room temperature for 1 hour. After the addition of water, the reaction mixture was extracted with ethyl acetate. The organic layer was washed two times with water, dried over anhydrous magnesium sulfate, and concentrated under reduced pressure. The obtained residue and 2 ml of 2-propyN-1-ol were mixed, and stirred at about 80°C for 30 minutes. The reaction mixture was cooled to room temperature, and extracted with ethyl acetate after the addition of water. The organic layer was successively washed with water, a saturated sodium bicarbonate aqueous solution, and a saturated sodium chloride aqueous solution; dried over anhydrous magnesium sulfate, and concentrated under reduced pressure. The residue was subjected to silica gel column chromatography to obtain 311 mg of N-3',4'-dimethoxybiphenyl-2-yl)-2-(propynyloxy)-2-(4-methylphenyl)acetamide (the present compound 1-063).

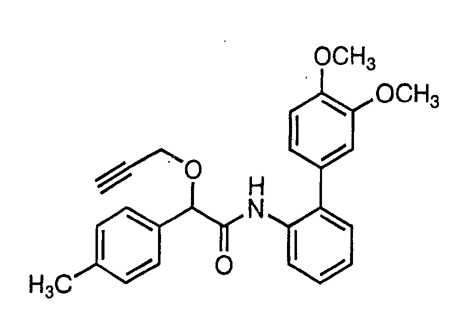

¹H-NMR (CDCl₃, TMS) d (ppm): 8.79 (1H, s), 8.38 (1H, d, J=8.4 Hz), 7.13-7.35 (7H, m), 7.01 (1H, d, J=8.0 Hz), 6.95 (1H, dd, J=8.4, 2.0 Hz), 6.90 (1H, d, J=2.0 Hz), 5.01 (1H, s), 3.88-4.05 (8H, m), 2.44 (1H, t, J=2.4 Hz), 2.33 (3H, s)

### Production Example 2

From 2-(4-ethylphenyl)-N-(3',4'-dihydroxybiphenyl-2-yl)-2-hydroxyacetamide and propargyl alcohol, N-(3',4'-dimethoxybiphenyl-2-yl)-2-(4-ethylphenyl)-2-(2-propynyloxy)acetamide (the present compound 1-064) was obtained by the similar method of Production Example 1.

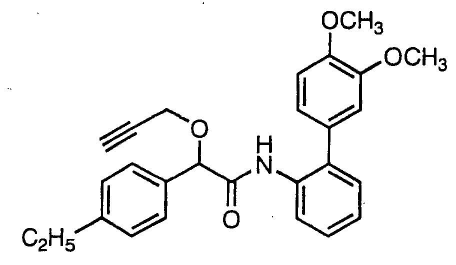

¹H-NMR (CDCl₃, TMS) d (ppm): 8.80 (1H, br), 8.38 (1H, dd, J=1.2, 8.3 Hz), 7.1-7.4 (7H, m), 7.00 (1H, d, J=8.0 Hz), 6.96 (1H, dd, J=2.0, 8.0 Hz), 6.91 (1H, d, J=2.0 Hz), 5.01 (1H, s), 4.07 (1H, dd, J=2.4, 15.8 Hz), 3.96 (3H, s), 3.90 (1H, dd, J=2.4, 15.6 Hz), 3.90 (3H, s), 2.63 (2H, q, J=7.7, 15.2 Hz), 2.44 (1H, t, J=2.4 Hz), 1.21 (3H, t, J=7.7 Hz)

### Production Example 3

From 2-(4-methoxyphenyl)-N-(3',4'-dimethoxybiphenyl-2-yl)-2-hydroxyacetamide and propargyl alcohol, N-(3',4'-dimethoxybiphenyl-2-yl)-2-(4-methoxyphenyl)-2-(2-propynyloxy)acetamide (the present compound 1-080) was obtained by the similar method of Production Example1.

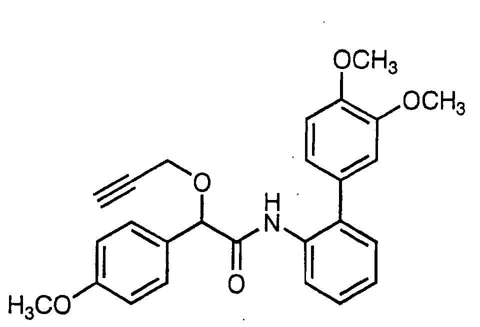

¹H-NMR (CDCl₃, TMS) d (ppm): 8.78 (1H, br), 8.38 (1H, d, J=8.7 Hz), 7.2-7.4 (4H, m), 7.1-7.2 (1H, m), 6.8-7.0 (5H, m), 4.99 (1H, s), 4.0-4.1 (1H, m), 3.95 (3H, s), 3.90 (3H, s), 3.8-3.9 (1H, m), 3.79 (3H, s), 2.44 (1H, t, J=2.4 Hz)

### Production Example 4

From 2-(4-methylphenyl)-N-(3',4'-dimethoxy-4-methylbiphenyl-2-yl)-2-hydroxyacetamide and propargyl alcohol, N-(3',4'-dimethoxy-4-methylbiphenyl-2-yl)-2-(2-propynyloxy)-2-(4-methylphenyl)acetamide (the present compound 2-078) was obtained by the similar method of Production Example1.

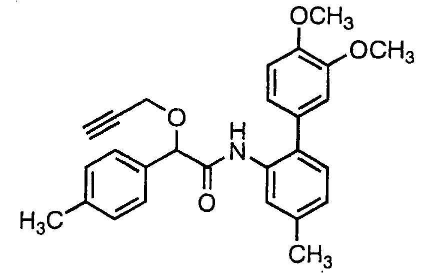

¹H-NMR (CDCl₃, TMS) d (ppm): 8.75 (1H, br), 8.23 (1H, s), 7.1-7.3 (5H, m), 6.8-7.0 (4H, m), 5.00 (1H, s), 4.06 (1H, dd, J=2.4, 15.8 Hz), 3.94 (3H, s), 3.90 (1H, dd, J=2.2, 15.6 Hz), 3.89 (3H, s), 2.44 (1H, t, J=2.4 Hz), 2.35 (3H, s), 2.32 (3H, s)

### Production Example 5

From 2-(3-methylphenyl)-n-(3',4'-dimethoxybiphenyl-2-yl)-2-hydroxyacetamide and propargyl alcohol, N-(3',4'-dimethoxybiphenyl-2-yl)-2-(2-propynyloxy)-2-(3-methylphenyl)acetamide (the present compound 1-098) was obtained by the similar method of Production Example1.

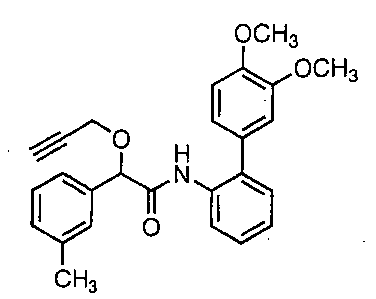

¹H-NMR (CDCl₃, TMS) d (ppm): 8.80 (1H, br), 8.28 (1H, d, J=8.3 Hz), 7.1-7.4 (5H, m), 7.00 (1H, d, J=8.3 Hz), 6.95 (1H, dd, J=1.9, 8.2 Hz), 6.91 (1H, d, J=1.9 Hz), 5.00 (1H, s), 4.08 (1H, dd, J=2.4, 15.6 Hz), 3.95 (3H, s), 3.90 (1H, dd, J=2.4, 15.7 Hz), 3.90 (3H, s), 2.4-2.5 (1H, m)

### Production Example 6

In 6 ml of N,N-dimethylformamide were added 0.47 g of N-(3'-methoxy-4'-hydroxybiphenyl-2-yl)-2-hyrdoxy-2-(4-methylphenyl)acetamide, 0.30 g of 3-bromo-1-propyn and 0.92 g of cesium carbonate, and the reaction mixture was stirred at 0 to 5°C for 1hour, at room temperature for 1hour, furthermore at 60°C for 3 hour. The reaction mixture was cooled to room temperature, and extracted with ethyl acetate after addition of water. The organic layer was washed with 5% hydrochloric acid, and saturated sodium chloride aqueous solution successively, dried over anhydrous magnesium sulfate, and concentrated under reduced pressure. The residue was subjected to silica gel column chromatography to obtain 225 mg of N-(3'-methoxy-4'-(2-propynyloxy)biphenyl-2-yl)-2-(2-propynyloxy)-2-(4-methylphenyl)a cetamide (the present compound 2-025).

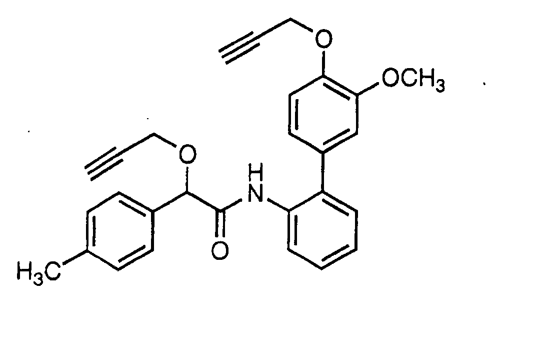

¹H-NMR (CDCl₃, TMS) d (ppm): 8.78 (1H, s), 8.38 (1H, d, J=8.1 Hz), 7.13-7.35 (8H, m), 6.92-6.94 (2H, m), 5.01 (1H, s), 3.88-4.10 (5H, m), 2.55-2.56 (1H, m), 2.45-2.46 (1H, m), 2.33 (3H, s)

### Production Example 7

In 15 ml of ethylene glycol dimethyl ether were added 1.20 g of N-(3',4'-dimethoxy-6-methylbiphenyl-2-yl)-2-hyrdoxy-2-(4-methylphenyl)acetamide, 1.22ml of 30% sodium hydroxide aqueous solution, 50 mg of tetrabutylammonium bromide and 543mg 3-bromopropyne / 5ml of toluene solution at room temperature, the mixture was stirred at 40°C for 6 hours. The reaction mixture was cooled to room temperature, and extracted with ethyl acetate after addition of water. The organic layer was washed with saturated sodium chloride aqueous solution, dried over anhydrous magnesium sulfate, and concentrated under reduced pressure. The residue was subjected to silica gel column chromatography to obtain 833 mg of N-(3',4'-dimethoxy-6-methylbiphenyl-2-yl)-2-(2-propynyloxy)-2-(4-methylphenyl)acetamide (the present compound 2-046).

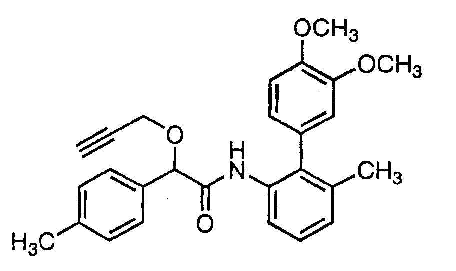

¹H-NMR (CDCl₃, TMS) d (ppm): 8.39 and 8.34 (1H in all, br), 8.1-8.3 (1H, m), 7.0-7.3 (7H, m), 6.6-6.9 (2H, m), 4.89 and 4.88 (1H in all, s), 3.8-4.0 (8H, m), 2.4-2.5 (1H, m), 2.32 and 2.31 (3H in all, s), 2.08 (3H, s)

### Production Example 8

In 5 ml of N,N-dimethylformamide were added 500 mg of N-(3',4'-dimethoxy-5-methyl-biphenyl-2-yl)-2-hyrdoxy-2-(4-methylphenyl)acetamide, 667 mg of cesium carbonate and 226 mg of 3-bromopropyne, the mixture was stirred at 60°C for 3.5 hours. The reaction mixture was cooled to room temperature, and extracted with ethyl acetate after addition of water. The organic layer was washed with saturated sodium chloride aqueous solution, dried over anhydrous magnesium sulfate, and concentrated under reduced pressure. The residue was subjected to silica gel column chromatography to obtain 130 mg of N-(3',4'-dimethoxy-5-methyl-biphenyl-2-yl)-2-(2-propynyloxy)-2-(4-methylphenyl)acetamide (the present compound 2-062).

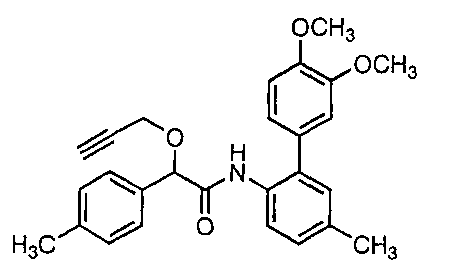

¹H-NMR (CDCl₃, TMS) d (ppm): 8.68 (1H, br), 8.22 (1H, d, J=8.3 Hz), 7.0-7.3 (6H, m), 6.98 (1H, d, J=8.3 Hz), 6.93 (1H, dd, J=1.8, 8.2 Hz), 6.88 (1H, d, J=1.9 Hz), 4.99 (1H, s), 4.06 (1H, dd, J=2.4, 15.8 Hz), 3.95 (3H, s), 3.89 (1H, dd, J=2.4, 15.6 Hz), 3.89 (3H, s), 2.43 (1H, t, J=2.2 Hz), 2.33 (3H, s), 2.32 (3H, s)

### Production Example 9

In 10 ml of tetrahydrofuran were added 0.70 g of 2-(4-chlorophenyl)-N-(3',4'-dihydroxybiphenyl-2-yl)-2-hydroxyacetamide and 231 mg of triethylamine, and 241 mg of methanesulfonyl chloride was added dropwise at about 0°C. The mixture was stirred at the same temperature for 30 minutes and at room temperature for 60 minutes. After addition of water, the reaction mixture was extracted with ethyl acetate. The organic layer was washed with 5% hydrochloric acid and saturated sodium chloride aqueous solution, dried over anhydrous magnesium sulfate, and concentrated under reduced pressure to obtain 3.43 g of a residue. 0.69 g of the obtained residue and 0.81 g 2-propyn-1-ol were mixed, and stirred at about 80°C for 2 hours. The reaction mixture was cooled to room temperature, and extracted with ethyl acetate after addition of water. The organic layer was washed with saturated sodium chloride aqueous solution, dried over anhydrous magnesium sulfate, and concentrated under reduced pressure. The residue was subjected to silica gel column chromatography to obtain 180 mg of 2-(4-chlorophenyl)-N-(3',4'-dihydroxybiphenyl-2-yl)-2-(2-propynyloxy)acetamide (the present compound 1-060).

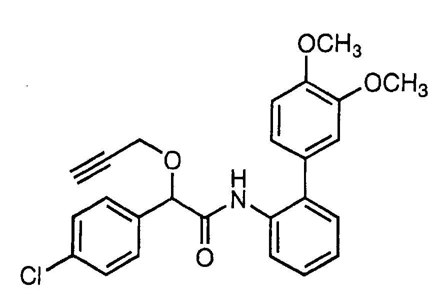

¹H-NMR (CDCl₃, TMS) d (ppm): 8.74 (1H, br), 8.33 (1H, d, J=8.4 Hz), 7.2-7.4 (6H, m), 7.1-7.2 (1H, m), 6.8-7.1 (3H, m), 5.02 (1H, s), 3.9-4.2 (2H, m), 3.96 (3H, s), 3.89 (3H, s), 2.46 (1H, t, J=2.3 Hz)

### Production Example 10

From 2-phenyl-N-(3',4'-dihydroxybiphenyl-2-yl)-2-hydroxyacetamide and propargyl alcohol, N-(3',4'-dimethoxybiphenyl-2-yl)-2-phenyl-2-(2-propynyloxy)acetamide (the present compound 1-058) was obtained by the similar method of Production Example 9.

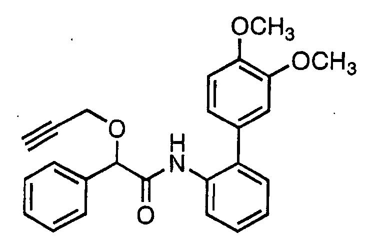

¹H-NMR (CDCl₃, TMS) d (ppm): 8.77 (1H, br), 8.37 (1H, dd, J=0.97, 8.3 Hz), 7.2-7.5 (7H, m), 7.1-7.2 (1H, m), 7.00 (1H, d, J=8.0 Hz), 6.94 (1H, dd, J=2.0, 8.3 Hz), 6.90 (1H, d, J=2.0 Hz), 5.03 (1H, s), 3.8-4.2 (2H, m), 3.96 (3H, s), 3.89 (3H, s), 2.45 (1H, t, J=2.4 Hz)

### Production Example 11

From 2-(4-chlorophenyl)-N-(3',4'-dihydroxybiphenyl-2-yl)-2-hydroxyacetamide and methanol, 2-(4-chlorophenyl)-N-(3',4'-dihydroxybiphenyl-2-yl)-2-methoxyacetamide (the present compound 1-003) was obtained by the similar method of Production Example 9.

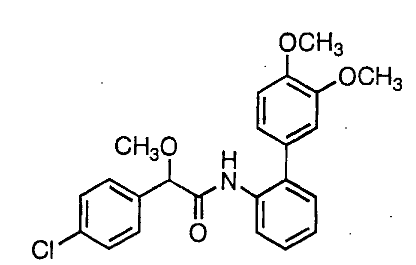

¹H-NMR (CDCl₃, TMS) d (ppm): 8.72 (1H, br), 8.31 (1H, d, J=8.0 Hz), 7.1-7.4 (7H, m), 6.98 (1H, d, J=7.8 Hz), 6.90 (1H, dd, J=2.0, 8.0 Hz), 6.87 (1H, d, J=1.9 Hz), 4.61 (1H, s), 3.97 (3H, s), 3.88 (3H, s), 3.25 (3H, s)

### Production Example 12

From 2-(4-fluorophenyl)-N-(3',4'-dihydroxybiphenyl-2-yl)-2-hydroxyacetamide and propargyl alcohol 2-(4-fluorophenyl)-N-(3',4'-dihydroxybiphenyl-2-yl)-2-(2-propynyloxy)acetamide (the present compound 1-059) was obtained by the similar method of Production Example 9.

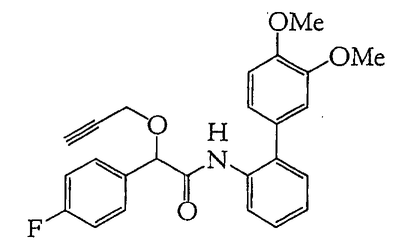

¹H-NMR (CDCl₃, TMS) d (ppm): 8.77 (1H, br), 8.30 (1H, dd, J=8.3, 0.97 Hz), 7.2-7.4 (4H, m), 7.1-7.2 (1H, m), 7.0-7.1 (3H, m), 6.93 (1H, dd, J=1.9, 8.3 Hz), 6.89 (1H, d, J=1.9 Hz), 5.02 (1H, s), 4.08 (1H, dd, J=2.4,15.6 Hz), 3.95 (3H, s), 3.91 (1H, dd, J=2.4, 15.6 Hz), 3.89 (3H, s), 2.46 (1H, t, J=2.4 Hz)

### Production Example 13

From 2-(4-chlorophenyl)-N-(3',4'-dihydroxybiphenyl-2-yl)-2-hydroxyacetamide ethyl alcohol, 2-(4-chlorophenyl)-N-(3',4'-dihydroxybiphenyl-2-yl)-2-ethoxyacetamide (the present compound 1-111) was obtained by the similar method of Production Example 9.

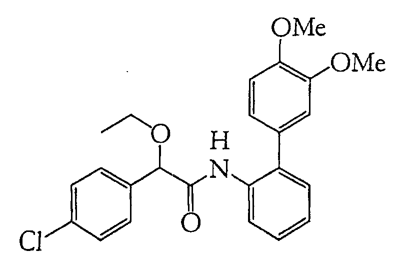

¹H-NMR (CDCl₃, TMS) d (ppm): 8.77 (1H, br), 8.30 (1H, dd, J=8.3, 0.97 Hz), 7.2-7.4 (4H, m), 7.1-7.2 (1H, m), 7.0-7.1 (3H, m), 6.93 (1H, dd, J=1.9, 8.3 Hz), 6.89 (1H, d, J=1.9 Hz), 5.02 (1H, s), 4.08 (1H, dd, J=2.4, 15.6 Hz), 3.95 (3H, s), 3.91 (1H, dd, J=2.4, 15.6 Hz), 3.89 (3H, s), 2.46 (1H, t, J=2.4 Hz)

### Production Example 14

From 2-(4-chlorophenyl)-N-(3',4'-dihydroxybiphenyl-2-yl)-2-hydroxyacetamide and isopropyl alcohol, 2-(4-chlorophenyl)-N-(3',4'-dihydroxybiphenyl-2-yl)-2-isopropoxyacetamide (the present compound 1-252) was obtained by the similar method of Production Example 9.

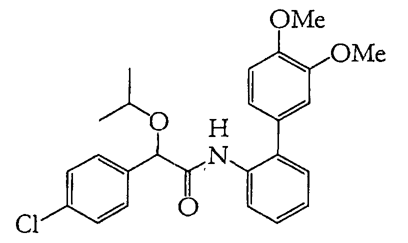

¹H-NMR (CDCl₃, TMS) d (ppm): 8.94 (1H, br), 8.39 (1H, d, J=8.3 Hz), 7.1-7.4 (7H, m), 6.8-7.1 (3H, m), 4.78 (1H, s), 3.95 (3H, s), 3.89 (3H, s), 3.5-3.6 (1H, m), 0.96 (3H, d, J=6.1 Hz), 0.92 (3H, d, J=6.0 Hz)

### Production Example 15

From 2-(4-chlorophenyl)-N-(3',4'-dihydroxybiphenyl-2-yl)-2-hydroxyacetamide and 2,2,2-trifluoroethyl alcohol, 2-(4-chlorophenyl)-N-(3',4'-dihydroxybiphenyl-2-yl)-2-(2,2,2-trifluoroethoxy)acetamide (the present compound 1-234) was obtained by the similar method of Production Example 9.

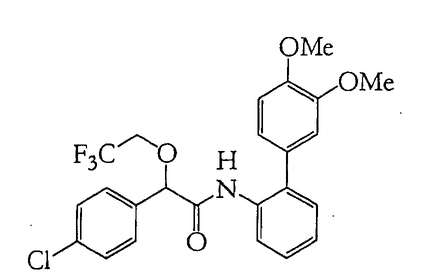

¹H-NMR (CDCl₃, TMS) d (ppm): 8.60 (1H, br), 8.31 (1H, d, J=7.5), 7.2-7.4 (6H, m), 7.1-7.2 (1H, m), 6.98 (1H, d, J=8.0 Hz), 6.90 (1H, dd, J=1.9, 8.0 Hz), 6.82 (1H, d, J=1.9 Hz), 4.87 (1H, s), 3.95 (3H, s), 3.87 (3H, s), 3.6-3.8 (2H, s)

### Production Example 16

From 2-(4-chlorophenyl)-N-(3',4'-dihydroxybiphenyl-2-yl)-2-hydroxyacetamide and 2-butyn-1-ol, 2-(4-chlorophenyl)-N-(3',4'-dihydroxybiphenyl-2-yl)-2-(2-butynyloxy)acetamide (the present compound 1-241) was obtained by the similar method of Production Example 9.

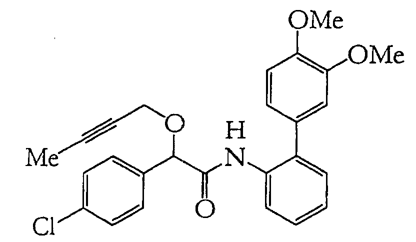

¹H-NMR (CDCl₃, TMS) d (ppm): 8.76 (1H, br), 8.34 (1H, d, J=8.4), 7.2-7.4 (6H, m), 7.1-7.2 (1H, m), 6.99 (1H, d, J=8.0 Hz), 6.93 (1H, dd, J=1.9, 8.0 Hz), 6.89 (1H, d, J=1.9 Hz), 5.03 (1H, s), 4.0-4.1 (1H, m), 3.96 (3H, s), 3.8-3.9 (1H, m), 3.90 (3H, s), 1.81 (3H, t, J=2.0 Hz)

### Production Example 17

In 5 ml of tetrahydrofuran were dissolved 398 mg of 2-(4-chlorophenyl)-N-(3',4'-dihydroxybiphenyl-2-yl)-2-hydroxyacetamide and 131 mg of triethylamine, 94 mg of acetyl chloride was added dropwise. The mixture was stirred at room temperature for 1 hour and 30 minutes. After addition of water, the reaction mixture was extracted with ethyl acetate. The organic layer was washed with 5% hydrochloric acid and saturated sodium chloride aqueous solution, dried over anhydrous magnesium sulfate, and concentrated under reduced pressure. The residue was washed with N-hexane, subjected to filtration, and dried to obtain 403 mg of 2-acetyloxy-2-(4-chlorophenyl)-N-(3',4'-dimethoxybiphenyl-2-yl)acetamide (the present compound 1-202)

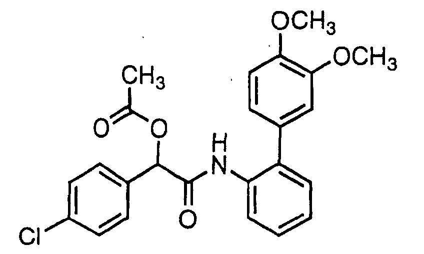

¹H-NMR (CDCl₃, TMS) d (ppm): 8.35 (1H, d, J=8.3 Hz), 8.06 (1H, br), 7.1-7.4 (7H, m), 6.96 (1H, d, J=8.0 Hz), 6.85 (1H, dd, J=1.8, 8.1 Hz), 6.82 (1H, d, J=1.7 Hz), 6.03 (1H, s), 3.95 (3H, s), 3.87 (3H, s), 1.92 (3H, s)

### Production Example 18

From 2-(4-chlorophenyl)-N-(3',4'-dimethoxybiphenyl-2-yl)-2-hydroxyacetamide and 2-methylpropionic chloride, 2-(4-chlorophenyl)-N-(3',4'-dihydroxybiphenyl-2-yl)-2-isobutyryloxyacetamide (the present compound 1-210) was obtained by the similar method of Production Example 12.

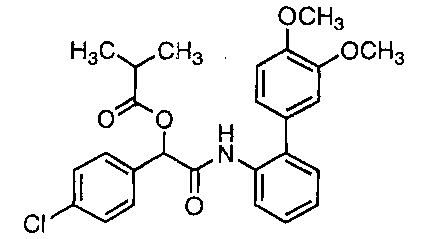

¹H-NMR (CDCl₃, TMS) d (ppm): 8.35 (1H, d, J=8.0 Hz), 8.09 (1H, br), 7.2-7.5 (7H, m), 7.1-7.4 (7H, m), 6.95 (1H, d, J=8.0 Hz), 6.86 (1H, dd, J=1.9, 8.0 Hz), 6.82 (1H, d, J=1.9 Hz), 6.03 (1H, s), 3.95 (3H, s), 3.86 (3H, s)2.3-2.4 (1H, m), 1.01 (6H, d, J=6.8 Hz)

### Production Example 19

From 2-(4-chlorophenyl)-N-(3',4'-dihydroxybiphenyl-2-yl)-2-hydroxyacetamide and 2,2-dimethylpropionic chloride, 403mg of 2-(4-chlorophenyl)-N-(3',4'-dihydroxybiphenyl-2-yl)-2-pyvaloyloxyacetamide (the present compound 1-214) was obtained by the similar method of Production Example 12.

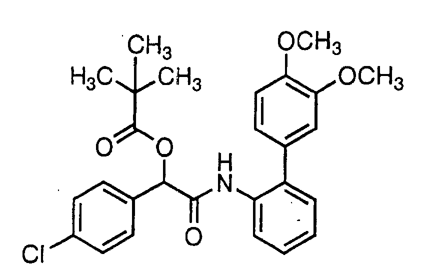

¹H-NMR (CDCl₃, TMS) d (ppm): 8.34 (1H, d, J=8.4 Hz), 7.98 (1H, br), 7.1-7.4 (7H, m)6.95 (1H, d, J=8.3 Hz), 6.86 (1H, dd, J=1.9, 8.3 Hz), 6.81 (1H, d, J=1.7 Hz), 6.03 (1H, s), 3.95 (3H, s), 3.84 (3H, s), 1.00 (9H, s)

### Production Example 27

In 30 ml of tetrahydrofuran were added 1.75 g of N-(3',4'-dimethoxybiphenyl-2-yl)-2-hyrdoxy-2-(4-methylphenyl)acetamide and 0.78 ml of triethylamine, and 0.38ml of methanesulfonyl chloride was added dropwise. The mixture was stirred at room temperature for 30 minutes. After addition of water, the reaction mixture was extracted with ethyl acetate. The organic layer was washed with water and saturated sodium chloride aqueous solution, dried over magnesium sulfate, and concentrated under reduced pressure. The obtained residue and 10ml of methanol were mixed, and stirred at about 65°C for 1 hour. The reaction mixture was cooled to room temperature, concentrated under reduced pressure, and extracted with ethyl acetate after addition of water. The organic layer was washed with water, dried over magnesium sulfate, and concentrated under reduced pressure. The residue was subjected to silica gel column chromatography to obtain 1.3 g of N-(3',4'-dimethoxybiphenyl-2-yl)-2-methoxy-2-(4-methylphenyl)acetamide (the present compound 1-006).

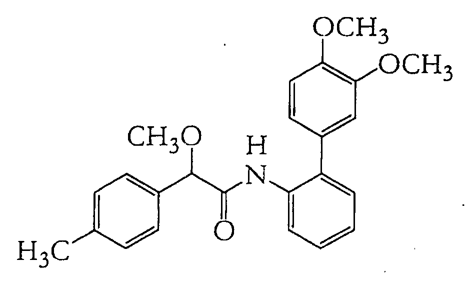

¹H-NMR (CDCl₃, TMS) d (ppm): 8.36 (1H, s), 8.35-8.37 (1H, m), 7.12-7.34 (7H, m), 6.99 (1H, d, J=8.0 Hz), 6.88-6.93 (2H, m), 4.60 (1H, s), 3.94 (3H, s), 3.89 (3H, s), 3.22 (3H, s), 2.33 (3H, s)

Next, the production examples of intermediate of the present compound are shown as the reference production example.

### Reference Production Example 1

In a mixture of 8.00 g of aluminum chloride and 80 ml of dichloromethane was added 7.85 g of ethyl oxalyl chloride dropwide at 0°C over stirring. After addition of 4.60 g of toluene at the same temperature, the mixture was stirred for 1 hour. The reaction mixture was poured in ice water, and extracted with chloroform. The organic layer was washed two times with saturated sodium chloride aqueous solution, dried over magnesium sulfate, and concentrated under reduced pressure to obtain 9.3 g of ethyl 2-(4-methylphenyl)-2-oxoacetate in crude form.

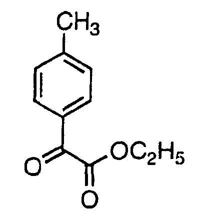

¹H-NMR (CDCl₃, TMS) d (ppm): 7.91 (2H, d, J=8.2 Hz), 7.31 (2H, d, J=8.1 Hz), 4.44 (2H, q, J=7.1 Hz), 2.44 (3H, s), 1.42 (2H, t, J=7.0 Hz)

### Reference Production Example2

9.3 g of ethyl 2-(4-methylphenyl)-2-oxoacetate, 13 ml of 30% sodium hydroxide aqueous solution and 15 ml of ethanol were mixed, and heated under reflux condition for 2 hours. The reaction mixture was cooled to room temperature, acidified by addition of 5% hydrochloric acid, and extracted with ethyl acetate. The organic layer was washed two times with saturated sodium chloride aqueous solution, dried over magnesium sulfate, and concentrated under reduced pressure. The residue was washed with hexane, dried to obtain 5.2g of 2-(4-methylphenyl)-2-oxoacetic acid.

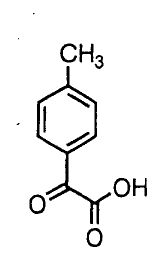

¹H-NMR (CDCl₃, TMS) d (ppm): 8.25 (2H, d, J=8.3 Hz), 7.32 (2H, d, J=8.0 Hz), 4.28 (1H, br), 2.45 (3H, s)

### Reference Production Example 3

In 20 ml of toluene were added 2.08 g of 2-(4-methylphenyl)-2-oxoacetic acid, 1.5 ml of thionyl chloride and 49mg of N,N-dimethylformamide, and stirred at 80°C for 30 minutes and at 100°C for 30 minutes. The reaction mixture was cooled to room temperature, concentrated under reduce pressure to obtain 2-(4-methylphenyl)-2-oxoacetic chloride. The obtained 2-(4-methylphenyl)-2-oxoacetic chloride was added at 0 to 5°C in the solution dissolved 2.91 g of 3',4'-dimethoxy-2-aminobiphenyl and 2.6 ml of triethylamine in 30 ml of tetrahydrofuran, stirred at room temperature for 2 hours. After addition of water, the reaction mixture was extracted with ethyl acetate. The organic layer was washed with 5% hydrochloric acid, saturated sodium bicarbonate aqueous solution and saturated sodium chloride aqueous solution successively, dried over magnesium sulfate, and concentrated under reduced pressure to obtain 3.57 g of N-(3',4'-dimethoxybiphenyl-2-yl)-2-(4-methylphenyl)-2-oxoacetamide.

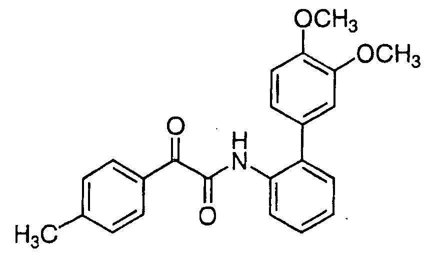

¹H-NMR (CDCl₃, TMS) d (ppm): 9.28 (1H, s), 8.56 (1H, d, J=7.2 Hz), 8.29 (2H, d, J=8.4 Hz), 7.40-7.44 (m, 1H), 7.22-7.35 (4H, m), 6.93-7.02 (m, 3H), 3.96 (3H, s), 3.91 (3H, s), 2.43 (3H, s)

### Reference Production Example 4

In 10 ml of ethanol was dissolved 0.60 g of N-(3',4'-dimethoxybiphenyl-2-yl)-2-(4-methylphenyl)-2-oxoacetamide, and 0.12g of sodium borohydride was added. The mixture was stirred at room temperature for 4 hours, and then extracted with ethyl acetate after addition of water and 5% hydrochloric acid. The organic layer was washed with water, saturated sodium bicarbonate aqueous solution and saturated sodium chloride aqueous solution successively, dried over magnesium sulfate, and concentrated under reduced pressure. The residue was subjected to silica gel column chromatography to obtain 285 mg of N-(3',4'-dimethoxybiphenyl-2-yl)-2-hyrdoxy-2-(4-methylphenyl)acetamide.

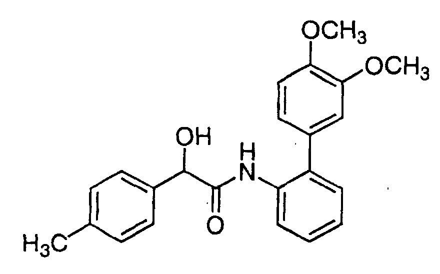

¹H-NMR (CDCl₃, TMS) d (ppm): 8.37 (1H, d, J=4.4 Hz), 7.91 (1H, s), 7.32-7.37 (1H, m), 7.01-7.26 (m, 6H), 6.85 (1H, d, J=8.0 Hz), 6.70 (1H, dd, J=8.0, 2.0 Hz), 6.66 (1H, d, J=2.0 Hz), 4.98 (1H, d, J=3 Hz), 3.96 (3H, s), 3.79 (3H, s), 3.51 (1H, d, J=3 Hz), 2.34 (3H, s)

### Reference Production Example 5

In 150 ml of toluene were added 15.0 g of 2-(4-methylphenyl)-2-oxoacetic acid, 10.7 ml of thionyl chloride and 0.3 ml of N,N-dimethylformamide, and stirred at 80°C for 30 minutes and at 100°C for 30 minutes. The reaction mixture was cooled to room temperature, and concentrated under reduced pressure to obtain 2-(4-methylphenyl)-2-oxoacetic chloride in crude form. Another hand, in 150 ml of tetrahydrofuran were dissolved 15.7g of 2-bromaniline and 19 ml of triethylamine, and the 2-(4-methylphenyl)-2-oxoacetic chloride was added at 0 to 5°C. The mixture was stirred at room temperature for 3 hours. After addition of water, the reaction mixture was extracted with ethyl acetate. The organic layer was washed with 5% hydrochloric acid, saturated sodium bicarbonate aqueous solution and saturated sodium chloride aqueous solution successively, dried over magnesium sulfate, and concentrated under reduced pressure. The residue was washed with hexane to obtain 21.9 g of N-(2-bromophenyl)-2-(4-methylphenyl)-2-oxoacetamide.

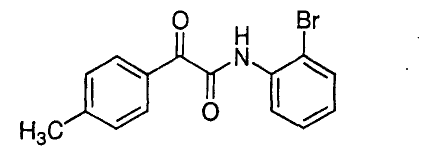

¹H-NMR (CDCl₃, TMS) d (ppm): 9.60 (1H, s), 8.51 (1H, dd, J=8.4, 1.2 Hz), 8.39 (2H, d, J=8.4 Hz), 7.61 (1H, dd, J=8.0, 1.2 Hz), 7.31-7.41 (3H, m), 7.04-7.26 (m, 1H), 2.46 (3H, s)

### Reference Production Example 6

In 100 ml of ethanol were added 10.0 g of N-(2-bromophenyl)-2-(4-methylphenyl)-2-oxoacetamide and 0.36 g sodium borohydride, and stirred at room temperature for 8 hours. After addition of saturated ammonium chloride aqueous solution, the reaction mixture was concentrated under reduced pressure, and extracted with ethyl acetate. The organic layer was washed with saturated sodium chloride aqueous solution, dried over magnesium sulfate, and concentrated to obtain 10.56 g of N-(2-bromophenyl)-2-hyrdoxy-2-(4-methylphenyl)acetamide.

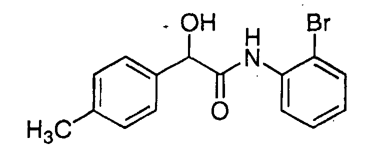

¹H-NMR (CDCl₃, TMS) d (ppm): 8.70 (1H, s), 8.36 (1H, dd, J=8.4, 1.6 Hz), 7.52 (1H, dd, J=8.0, 1.2 Hz), 7.40 (2H, d, J=8.0 Hz), 7.19-7.32 (3H, m), 6.96-7.00 (m, 1H), 5.22 (1H, d, J=1.6 Hz), 3.30 (1H, s), 2.36 (3H, s)

### Reference Production Example 7

In 25 ml of ethylene glycol dimethyl ether were added 2.33 g of N-(2-bromophenyl)-2-hyrdoxy-2-(4-methylphenyl)acetamide, 2.0 g of 2-methoxy-4-(4,4,5,5-tetramethyl-1,3,2-dioxaboran-2-yl)phenol, 4.6 g of potassium phosphate hydrate and 0.18 g of {1,1'-bis(diphenylphosphino)ferrocene}palladium(II) dichloride(II) methylene chloride complex, and stirred under nitrogen atmosphere at 80°C for 3 hours. The reaction mixture was cooled at room temperature, and filtered. The filtrate was concentrated under reduced pressure. The residue was subjected to silica gel column chromatography to obtain 2.6 g of N-(3'-methoxy-4'-hydroxybiphenyl-2-yl)-2-hyrdoxy-2-(4-methylphenyl)acetamide.

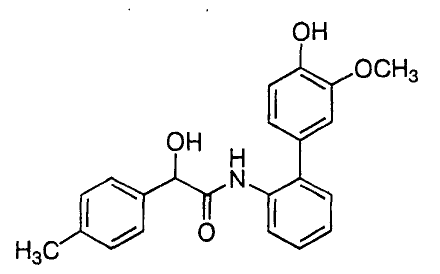

¹H-NMR (CDCl₃, TMS) d (ppm): 8.36 (1H, d, J=8.4 Hz), 7.88 (1H, s), 7.32-7.35 (1H, m), 7.01-7.26 (m, 6H), 6.88 (1H, d, J=7.6 Hz), 6.61-6.64 (2H, m), 5.70 (1H, s), 4.98 (1H, d, J=2.8 Hz), 3.79 (3H, s), 3.61-3.63 (1H, m), 2.35 (3H, s)

### Reference Production Example 8

In 140 ml of tetrahydrofuran were added 40.0 g of 4-bromochlorobenzene and 5.33 g of magnesium under nitrogen atmosphere, and stirred to prepare Grignard reagent. In 750 ml of tetrahydrofuran was dissolved 49.3 g of diethyl oxalyl, and the Grignard reagent was added dropwise at -70°C over 30 minutes. The reaction mixture was warmed to room temperature over 2 hours, and stirred at room temperature for 2 hours. After addition of ice water and saturated ammonium chloride aqueous solution, the organic solvent was removed by distillation under reduce pressure from the reaction mixture. The residue was filtered, and the filtrate was extracted with ethyl acetate. The organic layer was washed with saturated sodium chloride aqueous solution, dried over magnesium sulfate, and concentrated under reduced pressure. The residue was subjected to silica gel column chromatography to obtain 22.1 g of ethyl 2-(4-chlorophenyl)-2-oxoacetate.

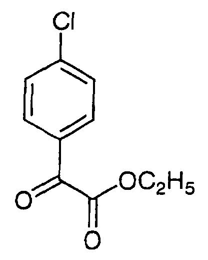

¹H-NMR (CDCl₃, TMS) d (ppm): 7.98-8.01 (2H, m), 7.48-7.51 (2H, m), 3.98 (3H, s)

### Reference Production Example 9

In 300 ml of ethanol were added 12.2 g of 2-(4-chlorophenyl)-2-oxoethyl acetate and 12.6 g of 20% sodium hydroxide aqueous solution, and stirred at room temperature for 3 hours. The reaction mixture was acidified by addition of 36% hydrochloric acid, and extracted three times with chloroform. The organic layer was dried over anhydrous magnesium sulfate, and concentrated under reduced pressure. The residue was washed with hexane, and dried to obtain 5.2 g of 2-(4-chlorophenyl)-2-oxoacetic acid.

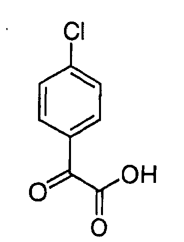

¹H-NMR (CDCl₃, TMS) d (ppm): 8.36-8.39 (1H, m), 8.0 (1H, br), 7.51-7.54 (2H, m)

### Reference Production Example 10

In 12 ml of 1,4-dioxane were dissolved 1.12 g of 2-(4-chlorophenyl)-2-oxoacetic acid and 3 ml of N,N-dimethylformamide, and 1.18 g of 1,1'-carbonyldiimidazole was added at about 0°C over stirring. After the mixture was stirred for 1.5 hours, 1.39 g of 3',4'-dimethoxybiphenyl-2-ylamine was added dropwise in the mixture, and stirred at 0°C for 30 minutes and at room temperature for 2 hours. The reaction mixture was extracted two times with ethyl acetate after addition of water. The organic layer was washed with 5% hydrochloric acid and saturated sodium chloride aqueous solution, dried over magnesium sulfate, and concentrated under reduced pressure. The residue was washed with N-hexane, subjected to filtration, and dried to obtain 1.44 g of 2-(4-chlorophenyl)-N-(3',4'-dimethoxybiphenyl-2-yl)-2-oxoacetamide.

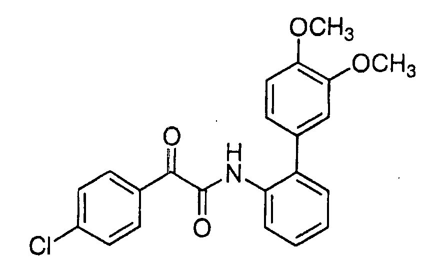

¹H-NMR (CDCl₃, TMS) d (ppm): 9.30 (1H, br), 8.53 (1H, d, J=8.3 Hz), 8.36 (2H, d, J=8.5 Hz), 7.2-7.4 (5H, m), 6.9-7.1 (3H, m), 3.96 (3H, s), 3.91 (3H, s)

### Reference Production Example 11

In 15 ml of methanol were dissolved 1.44 g of 2-(4-chlorophenyl)-N-(3',4'-dimethoxybiphenyl-2-yl)-2-oxoacetamide, and 69mg of sodium borohydride was added at about 0°C. After stirring for 1.5 hour, the reaction mixture was concentrated under reduced pressure. The obtained residue was extracted with ethyl acetate after addition of water. The organic layer was washed with 5% hydrochloric acid and saturated sodium chloride aqueous solution (two times) successively, dried over anhydrous magnesium sulfate, and concentrated under reduced pressure. The residue was subjected to silica gel column chromatography, and the obtained solid was washed with hexane to obtain 0.70 g of 2-(4-chlorophenyl)-N-(3',4'-dimethoxybiphenyl-2-yl)-2-hydroxyacetamide.

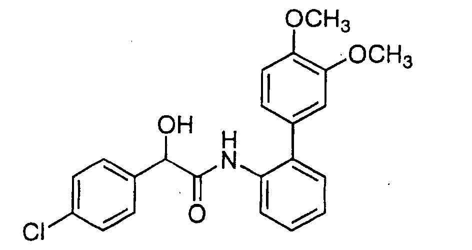

¹H-NMR (CDCl₃, TMS) d (ppm): 8.33 (1H, d, J=8.3 Hz), 7.95 (1H, br), 7.1-7.4 (7H, m), 6.83 (1H, d, J=8.0 Hz), 6.71 (1H, d, J=1.9 Hz), 6.65 (1H, dd, J=2.0, 7.9 Hzm), 4.99 (1H, br), 3.96 (3H, s), 3.82 (3H, s), 3.70 (1H, br)

### Reference Production Example 12

In 100 ml of ethylene glycol dimethyl ether were added 10.1 g of 2-bromonitrobenzene, 10.0g of 3,4-dimethoxyphenylboric acid, 31.8 g of potassium phosphate hydrate and 817mg of {1,1'-bis(diphenylphosphino)ferrocene}palladium(II) dichloride(II) methylene chloride complex, and stirred under nitrogen atmosphere at 80°C for 4.5 hours. The reaction mixture was cooled to room temperature, and subjected to filtration. The filtrate was concentrated under reduced pressure. The residue was subjected to silica gel column chromatography to obtain 10.0 g of 3,4-dimethoxy-2'-nitrobiphenyl.

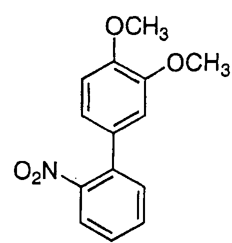

¹H-NMR (CDCl₃, TMS) d (ppm): 7.4-7.9 (4H, m), 6.92 (1H, d, J=8.3 Hz), 6.88 (1H, dd, J=1.9, 8.3 Hz), 6.83 (1H, d, J=1.9 Hz), 3.91 (3H, s), 3.88 (3H, s)

### Reference Production Example 13

In 100 ml of toluene were added 10.0 g of 3,4-dimethoxy-2'-nitrobiphenyl and 0.30g of 5% Pt/C, furthermore 5.74g of hydrazine hydrate was added at 80 to 100°C, and stirred at the same temperature for 2 hours. The reaction mixture was cooled at room temperature, and filtered with Celite (registered trade name) after addition of toluene and water. The filtrate was separated, and the organic layer was concentrated under reduced pressure. The residue was washed with hexane, and collected by filtration to obtain 8.47 g of 3',4'-dimethoxy-biphenyl-2-ylamine.

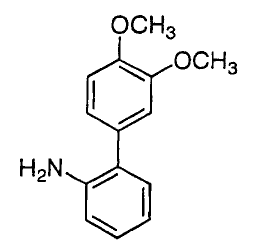

¹H-NMR (CDCl₃, TMS) d (ppm): 7.1-7.2 (2H, m), 6.9-7.1 (3H, m), 6.7-6.8 (2H, m), 3.92 (3H, s), 3.89 (3H, s), 3.77 (2H, br)

Next, examples of the present compound is shown with a numbers of the present compound.

The compound of the following formula`wherein R³, R⁴, R⁵, R¹¹, R¹², R¹³, R¹⁴, R¹⁵, R¹⁶ and R¹⁷ in the formula (1) are hydrogen atoms, and R¹, R², R⁶, R⁷, R⁸, R⁹, R¹⁰, X¹ and X² are the atom or group described in Table 1.

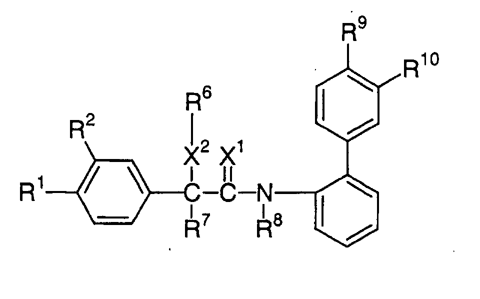

**Table 1**

| Number | R¹ | R² | R⁶ | R⁷ | R⁸ | R⁹ | R¹⁰ | X¹ | X² |
|---|---|---|---|---|---|---|---|---|---|
| | | | | | | | | | |
| 1-001 | H | H | CH₃ | H | H | OCH₃ | OCH₃ | O | O |
| 1-002 | F | H | CH₃ | H | H | OCH₃ | OCH₃ | O | O |
| 1-003 | Cl | H | CH₃ | H | H | OCH₃ | OCH₃ | O | O |
| 1-004 | Br | H | CH₃ | H | H | OCH₃ | OCH₃ | O | O |
| 1-005 | I | H | CH₃ | H | H | OCH₃ | OCH₃ | O | O |
| 1-006 | CH₃ | H | CH₃ | H | H | OCH₃ | OCH₃ | O | O |
| 1-007 | CH₂CH₃ | H | CH₃ | H | H | OCH₃ | OCH₃ | O | O |
| 1-008 | CH₂CH₂CH₃ | H | CH₃ | H | H | OCH₃ | OCH₃ | O | O |
| 1-009 | CH(CH₃)₂ | H | CH₃ | H | H | OCH₃ | OCH₃ | O | O |
| 1-010 | CH₂F | H | CH₃ | H | H | OCH₃ | OCH₃ | O | O |
| 1-011 | CHF₂ | H | CH₃ | H | H | OCH₃ | OCH₃ | O | O |
| 1-012 | CF₃ | H | CH₃ | H | H | OCH₃ | OCH₃ | O | O |
| 1-013 | CH₂CH₂F | H | CH₃ | H | H | OCH₃ | OCH₃ | O | O |
| 1-014 | CH₂CHF₂ | H | CH₃ | H | H | OCH₃ | OCH₃ | O | O |
| 1-015 | CH₂CF₃ | H | CH₃ | H | H | OCH₃ | OCH₃ | O | O |
| 1-016 | CHFCF₃ | H | CH₃ | H | H | OCH₃ | OCH₃ | O | O |
| 1-017 | CF₂CF₃ | H | CH₃ | H | H | OCH₃ | OCH₃ | O | O |
| 1-018 | CH=CH₂ | H | CH₃ | H | H | OCH₃ | OCH₃ | O | O |
| 1-019 | C(CH₃)=CH₂ | H | CH₃ | H | H | OCH₃ | OCH₃ | O | O |
| 1-020 | CH=CHCH₃ | H | CH₃ | H | H | OCH₃ | OCH₃ | O | O |
| 1-021 | C≡CH | H | CH₃ | H | H | OCH₃ | OCH₃ | O | O |
| 1-022 | C≡CCH₃ | H | CH₃ | H | H | OCH₃ | OCH₃ | O | O |
| 1-023 | OCH₃ | H | CH₃ | H | H | OCH₃ | OCH₃ | O | O |
| 1-024 | OCH₂CH₃ | H | CH₃ | H | H | OCH₃ | OCH₃ | O | O |
| 1-025 | OCH₂CH₂CH₃ | H | CH₃ | H | H | OCH₃ | OCH₃ | O | O |
| 1-026 | OCH(CH₃)₂ | H | CH₃ | H | H | OCH₃ | OCH₃ | O | O |
| 1-027 | OCH₂F | H | CH₃ | H | H | OCH₃ | OCH₃ | O | O |
| 1-028 | OCHF₂ | H | CH₃ | H | H | OCH₃ | OCH₃ | O | O |
| 1-029 | OCF₃ | H | CH₃ | H | H | OCH₃ | OCH₃ | O | O |
| 1-030 | SCH₃ | H | CH₃ | H | H | OCH₃ | OCH₃ | O | O |
| 1-031 | SCH₂CH₃ | H | CH₃ | H | H | OCH₃ | OCH₃ | O | O |
| 1-032 | SCH₂CH₂CH₃ | H | CH₃ | H | H | OCH₃ | OCH₃ | O | O |
| 1-033 | SCH(CH₃)₂ | H | CH₃ | H | H | OCH₃ | OCH₃ | O | O |
| 1-034 | SCH₂F | H | CH₃ | H | H | OCH₃ | OCH₃ | O | O |
| 1-035 | SCHF₂ | H | CH₃ | H | H | OCH₃ | OCH₃ | O | O |
| 1-036 | SCF₃ | H | CH₃ | H | H | OCH₃ | OCH₃ | O | O |
| 1-037 | c-Pr | H | CH₃ | H | H | OCH₃ | OCH₃ | O | O |
| 1-038 | H | F | CH₃ | H | H | OCH₃ | OCH₃ | O | O |
| 1-039 | H | Cl | CH₃ | H | H | OCH₃ | OCH₃ | O | O |
| 1-040 | H | Br | CH₃ | H | H | OCH₃ | OCH₃ | O | O |
| 1-041 | H | CH₃ | CH₃ | H | H | OCH₃ | OCH₃ | O | O |
| 1-042 | H | OCH₃ | CH₃ | H | H | OCH₃ | OCH₃ | O | O |
| 1-043 | Cl | F | CH₃ | H | H | OCH₃ | OCH₃ | O | O |
| 1-044 | Cl | Cl | CH₃ | H | H | OCH₃ | OCH₃ | O | O |
| 1-045 | Cl | Br | CH₃ | H | H | OCH₃ | OCH₃ | O | O |
| 1-046 | Cl | CH₃ | CH₃ | H | H | OCH₃ | OCH₃ | O | O |
| 1-047 | Cl | OCH₃ | CH₃ | H | H | OCH₃ | OCH₃ | O | O |
| 1-048 | Br | F | CH₃ | H | H | OCH₃ | OCH₃ | O | O |
| 1-049 | Br | Cl | CH₃ | H | H | OCH₃ | OCH₃ | O | O |
| 1-050 | Br | Br | CH₃ | H | H | OCH₃ | OCH₃ | O | O |
| 1-051 | Br | CH₃ | CH₃ | H | H | OCH₃ | OCH₃ | O | O |
| 1-052 | Br | OCH₃ | CH₃ | H | H | OCH₃ | OCH₃ | O | O |
| 1-053 | CH₃ | F | CH₃ | H | H | OCH₃ | OCH₃ | O | O |
| 1-054 | CH₃ | Cl | CH₃ | H | H | OCH₃ | OCH₃ | O | O |
| 1-055 | CH₃ | Br | CH₃ | H | H | OCH₃ | OCH₃ | O | O |
| 1-056 | CH₃ | CH₃ | CH₃ | H | H | OCH₃ | OCH₃ | O | O |
| 1-057 | CH₃ | OCH₃ | CH₃ | H | H | OCH₃ | OCH₃ | O | O |
| 1-058 | H | H | CH₂C≡CH | H | H | OCH₃ | OCH₃ | O | O |
| 1-059 | F | H | CH₂C≡CH | H | H | OCH₃ | OCH₃ | O | O |
| 1-060 | Cl | H | CH₂C≡CH | H | H | OCH₃ | OCH₃ | O | O |
| 1-061 | Br | H | CH₂C≡CH | H | H | OCH₃ | OCH₃ | 0 | O |
| 1-062 | I | H | CH₂C≡CH | H | H | OCH₃ | OCH₃ | O | O |
| 1-063 | CH₃ | H | CH₂C≡CH | H | H | OCH₃ | OCH₃ | O | O |
| 1-064 | CH₂CH₃ | H | CH₂C≡CH | H | H | OCH₃ | OCH₃ | O | O |
| 1-065 | CH₂CH₂CH₃ | H | CH₂C≡CH | H | H | OCH₃ | OCH₃ | O | O |
| 1-066 | CH(CH₃)₂ | H | CH₂C≡CH | H | H | OCH₃ | OCH₃ | O | O |
| 1-067 | CH₂F | H | CH₂C≡CH | H | H | OCH₃ | OCH₃ | O | O |
| 1-068 | CHF₂ | H | CH₂C≡CH | H | H | OCH₃ | OCH₃ | O | O |
| 1-069 | CF₃ | H | CH₂C≡CH | H | H | OCH₃ | OCH₃ | O | O |
| 1-070 | CH₂CH₂F | H | CH₂C≡CH | H | H | OCH₃ | OCH₃ | O | O |
| 1-071 | CH₂CHF₂ | H | CH₂C≡CH | H | H | OCH₃ | OCH₃ | O | O |
| 1-072 | CH₂CF₃ | H | CH₂C≡CH | H | H | OCH₃ | OCH₃ | O | O |
| 1-073 | CHFCF₃ | H | CH₂C≡CH | H | H | OCH₃ | OCH₃ | O | O |
| 1-074 | CF₂CF₃ | H | CH₂C≡CH | H | H | OCH₃ | OCH₃ | O | O |
| 1-075 | CH=CH₂ | H | CH₂C≡CH | H | H | OCH₃ | OCH₃ | O | O |
| 1-076 | C(CH₃)=CH₂ | H | CH₂C≡CH | H | H | OCH₃ | OCH₃ | O | O |
| 1-077 | CH=CHCH₃ | H | CH₂C≡CH | H | H | OCH₃ | OCH₃ | O | O |
| 1-078 | C≡CH | H | CH₂C≡CH | H | H | OCH₃ | OCH₃ | O | O |
| 1-079 | C≡CCH₃ | H | CH₂C≡CH | H | H | OCH₃ | OCH₃ | O | O |
| 1-080 | OCH₃ | H | CH₂C≡CH | H | H | OCH₃ | OCH₃ | O | O |
| 1-081 | OCH₂CH₃ | H | CH₂C≡CH | H | H | OCH₃ | OCH₃ | O | O |
| 1-082 | OCH₂CH₂CH₃ | H | CH₂C≡CH | H | H | OCH₃ | OCH₃ | O | O |
| 1-083 | OCH(CH₃)₂ | H | CH₂C≡CH | H | H | OCH₃ | OCH₃ | O | O |
| 1-084 | OCH₂F | H | CH₂C≡CH | H | H | OCH₃ | OCH₃ | O | O |
| 1-085 | OCHF₂ | H | CH₂C≡CH | H | H | OCH₃ | OCH₃ | O | O |
| 1-086 | OCF₃ | H | CH₂C≡CH | H | H | OCH₃ | OCH₃ | O | O |
| 1-087 | SCH₃ | H | CH₂C≡CH | H | H | OCH₃ | OCH₃ | O | O |
| 1-088 | SCH₂CH₃ | H | CH₂C≡CH | H | H | OCH₃ | OCH₃ | O | O |
| 1-089 | SCH₂CH₂CH₃ | H | CH₂C≡CH | H | H | OCH₃ | OCH₃ | O | O |
| 1-090 | SCH(CH₃)₂ | H | CH₂C≡CH | H | H | OCH₃ | OCH₃ | O | O |
| 1-091 | SCH₂F | H | CH₂C≡CH | H | H | OCH₃ | OCH₃ | O | O |
| 1-092 | SCHF₂ | H | CH₂C≡CH | H | H | OCH₃ | OCH₃ | O | O |
| 1-093 | SCF₃ | H | CH₂C≡CH | H | H | OCH₃ | OCH₃ | O | O |
| 1-094 | c-Pr | H | CH₂C≡CH | H | H | OCH₃ | OCH₃ | O | O |
| 1-095 | H | F | CH₂C≡CH | H | H | OCH₃ | OCH₃ | O | O |
| 1-096 | H | Cl | CH₂C≡CH | H | H | OCH₃ | OCH₃ | O | O |
| 1-097 | H | Br | CH₂C≡CH | H | H | OCH₃ | OCH₃ | O | O |
| 1-098 | H | CH₃ | CH₂C≡CH | H | H | OCH₃ | OCH₃ | O | O |
| 1-099 | H | OCH₃ | CH₂C≡CH | H | H | OCH₃ | OCH₃ | O | O |
| 1-100 | Cl | F | CH₂C≡CH | H | H | OCH₃ | OCH₃ | O | O |
| 1-101 | Cl | Cl | CH₂C≡CH | H | H | OCH₃ | OCH₃ | O | O |
| 1-102 | Cl | Br | CH₂C≡CH | H | H | OCH₃ | OCH₃ | O | O |
| 1-103 | Cl | CH₃ | CH₂C≡CH | H | H | OCH₃ | OCH₃ | O | O |
| 1-104 | Cl | OCH₃ | CH₂C≡CH | H | H | OCH₃ | OCH₃ | O | O |
| 1-105 | Br | F | CH₂C=CH | H | H | OCH₃ | OCH₃ | O | O |
| 1-106 | Br | Cl | CH₂C≡CH | H | H | OCH₃ | OCH₃ | O | O |
| 1-107 | Br | Br | CH₂C≡CH | H | H | OCH₃ | OCH₃ | O | O |
| 1-108 | Br | CH₃ | CH₂C≡CH | H | H | OCH₃ | OCH₃ | O | O |
| 1-109 | Br | OCH₃ | CH₂C≡CH | H | H | OCH₃ | OCH₃ | O | O |
| 1-110 | H | H | CH₂CH₃ | H | H | OCH₃ | OCH₃ | O | O |
| 1-111 | Cl | H | CH₂CH₃ | H | H | OCH₃ | OCH₃ | O | O |
| 1-112 | Br | H | CH₂CH₃ | H | H | OCH₃ | OCH₃ | O | O |
| 1-113 | CH₃ | H | CH₂CH₃ | H | H | OCH₃ | OCH₃ | O | O |
| 1-114 | CH₂CH₃ | H | CH₂CH₃ | H | H | OCH₃ | OCH₃ | O | O |
| 1-115 | OCH₃ | H | CH₂CH₃ | H | H | OCH₃ | OCH₃ | O | O |
| 1-116 | CF₃ | H | CH₂CH₃ | H | H | OCH₃ | OCH₃ | O | O |
| 1-117 | H | H | CH₂CH₂CH₃ | H | H | OCH₃ | OCH₃ | O | O |
| 1-118 | Cl | H | CH₂CH₂CH₃ | H | H | OCH₃ | OCH₃ | O | O |
| 1-119 | Br | H | CH₂CH₂CH₃ | H | H | OCH₃ | OCH₃ | O | O |
| 1-120 | CH₃ | H | CH₂CH₂CH₃ | H | H | OCH₃ | OCH₃ | O | O |
| 1-121 | CH₂CH₃ | H | CH₂CH₂CH₃ | H | H | OCH₃ | OCH₃ | O | O |
| 1-122 | OCH₃ | H | CH₂CH₂CH₃ | H | H | OCH₃ | OCH₃ | O | O |
| 1-123 | CF₃ | H | CH₂CH₂CH₃ | H | H | OCH₃ | OCH₃ | O | O |
| 1-124 | H | H | CH₂F | H | H | OCH₃ | OCH₃ | O | O |
| 1-125 | Cl | H | CH₂F | H | H | OCH₃ | OCH₃ | O | O |
| 1-126 | Br | H | CH₂F | H | H | OCH₃ | OCH₃ | O | O |
| 1-127 | CH₃ | H | CH₂F | H | H | OCH₃ | OCH₃ | O | O |
| 1-128 | CH₂CH₃ | H | CH₂F | H | H | OCH₃ | OCH₃ | O | O |
| 1-129 | OCH₃ | H | CH₂F | H | H | OCH₃ | OCH₃ | O | O |
| 1-130 | CF₃ | H | CH₂F | H | H | OCH₃ | OCH₃ | O | O |
| 1-131 | H | H | CHF₂ | H | H | OCH₃ | OCH₃ | O | O |
| 1-132 | Cl | H | CHF₂ | H | H | OCH₃ | OCH₃ | O | O |
| 1-133 | Br | H | CHF₂ | H | H | OCH₃ | OCH₃ | O | O |
| 1-134 | CH₃ | H | CHF₂ | H | H | OCH₃ | OCH₃ | O | O |
| 1-135 | CH₂CH₃ | H | CHF₂ | H | H | OCH₃ | OCH₃ | O | O |
| 1-136 | OCH₃ | H | CHF₂ | H | H | OCH₃ | OCH₃ | O | O |
| 1-137 | CF₃ | H | CHF₂ | H | H | OCH₃ | OCH₃ | O | O |
| 1-138 | H | H | CF₃ | H | H | OCH₃ | OCH₃ | O | O |
| 1-139 | Cl | H | CF₃ | H | H | OCH₃ | OCH₃ | O | O |
| 1-140 | Br | H | CF₃ | H | H | OCH₃ | OCH₃ | O | O |
| 1-141 | CH₃ | H | CF₃ | H | H | OCH₃ | OCH₃ | O | O |
| 1-142 | CH₂CH₃ | H | CF₃ | H | H | OCH₃ | OCH₃ | O | O |
| 1-143 | OCH₃ | H | CF₃ | H | H | OCH₃ | OCH₃ | O | O |
| 1-144 | CF₃ | H | CF₃ | H | H | OCH₃ | OCH₃ | O | O |
| 1-145 | H | H | CH₂C=CH₂ | H | H | OCH₃ | OCH₃ | O | O |
| 1-146 | Cl | H | CH₂C=CH₂ | H | H | OCH₃ | OCH₃ | O | O |
| 1-147 | Br | H | CH₂C=CH₂ | H | H | OCH₃ | OCH₃ | O | O |
| 1-148 | CH₃ | H | CH₂C=CH₂ | H | H | OCH₃ | OCH₃ | O | O |
| 1-149 | CH₂CH₃ | H | CH₂C=CH₂ | H | H | OCH₃ | OCH₃ | O | O |
| 1-150 | OCH₃ | H | CH₂C=CH₂ | H | H | OCH₃ | OCH₃ | O | O |
| 1-151 | CF₃ | H | CH₂C=CH₂ | H | H | OCH₃ | OCH₃ | O | O |
| 1-152 | H | H | CH₃ | H | CH₃ | OCH₃ | OCH₃ | O | O |
| 1-153 | Cl | H | CH₃ | H | CH₃ | OCH₃ | OCH₃ | O | O |
| 1-154 | Br | H | CH₃ | H | CH₃ | OCH₃ | OCH₃ | O | O |
| 1-155 | CH₃ | H | CH₃ | H | CH₃ | OCH₃ | OCH₃ | O | O |
| 1-156 | CH₂CH₃ | H | CH₃ | H | CH₃ | OCH₃ | OCH₃ | O | O |
| 1-157 | OCH₃ | H | CH₃ | H | CH₃ | OCH₃ | OCH₃ | O | O |
| 1-158 | CF₃ | H | CH₃ | H | CH₃ | OCH₃ | OCH₃ | O | O |
| 1-159 | H | H | CH₂C≡CH | H | CH₃ | OCH₃ | OCH₃ | O | O |
| 1-160 | Cl | H | CH₂C≡CH | H | CH₃ | OCH₃ | OCH₃ | O | O |
| 1-161 | Br | H | CH₂C≡CH | H | CH₃ | OCH₃ | OCH₃ | O | O |
| 1-162 | CH₃ | H | CH₂C≡CH | H | CH₃ | OCH₃ | OCH₃ | O | O |
| 1-163 | CH₂CH₃ | H | CH₂C≡CH | H | CH₃ | OCH₃ | OCH₃ | O | O |
| 1-164 | OCH₃ | H | CH₂C≡CH | H | CH₃ | OCH₃ | OCH₃ | O | O |
| 1-165 | CF₃ | H | CH₂C≡CH | H | CH₃ | OCH₃ | OCH₃ | O | O |
| 1-166 | H | H | CH₃ | H | H | OCH₃ | OCH₃ | S | O |
| 1-167 | Cl | H | CH₃ | H | H | OCH₃ | OCH₃ | S | O |
| 1-168 | Br | H | CH₃ | H | H | OCH₃ | OCH₃ | S | O |
| 1-169 | CH₃ | H | CH₃ | H | H | OCH₃ | OCH₃ | S | O |
| 1-170 | CH₂CH₃ | H | CH₃ | H | H | OCH₃ | OCH₃ | S | O |
| 1-171 | OCH₃ | H | CH₃ | H | H | OCH₃ | OCH₃ | S | O |
| 1-172 | CF₃ | H | CH₃ | H | H | OCH₃ | OCH₃ | S | O |
| 1-173 | H | H | CH₂C≡CH | H | H | OCH₃ | OCH₃ | S | O |
| 1-174 | Cl | H | CH₂C≡CH | H | H | OCH₃ | OCH₃ | S | O |
| 1-175 | Br | H | CH₂C≡CH | H | H | OCH₃ | OCH₃ | S | O |
| 1-176 | CH₃ | H | CH₂C≡CH | H | H | OCH₃ | OCH₃ | S | O |
| 1-177 | CH₂CH₃ | H | CH₂C≡CH | H | H | OCH₃ | OCH₃ | S | O |
| 1-178 | OCH₃ | H | CH₂C≡CH | H | H | OCH₃ | OCH₃ | S | O |
| 1-179 | CF₃ | H | CH₂C≡CH | H | H | OCH₃ | OCH₃ | S | O |
| 1-180 | H | H | CH₃ | H | H | OCH₃ | OCH₃ | O | S |
| 1-181 | Cl | H | CH₃ | H | H | OCH₃ | OCH₃ | O | S |
| 1-182 | Br | H | CH₃ | H | H | OCH₃ | OCH₃ | O | S |
| 1-183 | CH₃ | H | CH₃ | H | H | OCH₃ | OCH₃ | O | S |
| 1-184 | CH₂CH₃ | H | CH₃ | H | H | OCH₃ | OCH₃ | O | S |
| 1-185 | OCH₃ | H | CH₃ | H | H | OCH₃ | OCH₃ | O | S |
| 1-186 | CF₃ | H | CH₃ | H | H | OCH₃ | OCH₃ | O | S |
| 1-187 | H | H | CH₂C≡CH | H | H | OCH₃ | OCH₃ | O | S |
| 1-188 | Cl | H | CH₂C≡CH | H | H | OCH₃ | OCH₃ | O | S |
| 1-189 | Br | H | CH₂C≡CH | H | H | OCH₃ | OCH₃ | O | S |
| 1-190 | CH₃ | H | CH₂C≡CH | H | H | OCH₃ | OCH₃ | O | S |
| 1-191 | CH₂CH₃ | H | CH₂C≡CH | H | H | OCH₃ | OCH₃ | O | S |
| 1-192 | OCH₃ | H | CH₂C≡CH | H | H | OCH₃ | OCH₃ | O | S |
| 1-193 | CF₃ | H | CH₂C≡CH | H | H | OCH₃ | OCH₃ | O | S |
| 1-194 | H | H | CH₂C≡CH | CH₃ | H | OCH₃ | OCH₃ | O | O |
| 1-195 | Cl | H | CH₂C≡CH | CH₃ | H | OCH₃ | OCH₃ | O | O |
| 1-196 | Br | H | CH₂C=CH | CH₃ | H | OCH₃ | OCH₃ | O | O |
| 1-197 | CH₃ | H | CH₂C≡CH | CH₃ | H | OCH₃ | OCH₃ | O | O |
| 1-198 | CH₂CH₃ | H | CH₂C≡CH | CH₃ | H | OCH₃ | OCH₃ | O | O |
| 1-199 | OCH₃ | H | CH₂C=CH | CH₃ | H | OCH₃ | OCH₃ | O | O |
| 1-200 | CF₃ | H | CH₂C≡CH | CH₃ | H | OCH₃ | OCH₃ | O | O |
| 1-201 | H | H | COCH₃ | H | H | OCH₃ | OCH₃ | O | O |
| 1-202 | Cl | H | COCH₃ | H | H | OCH₃ | OCH₃ | O | O |
| 1-203 | Br | H | COCH₃ | H | H | OCH₃ | OCH₃ | O | O |
| 1-204 | CH₃ | H | COCH₃ | H | H | OCH₃ | OCH₃ | O | O |
| 1-205 | H | H | COCH₂CH₃ | H | H | OCH₃ | OCH₃ | O | O |
| 1-206 | Cl | H | COCH₂CH₃ | H | H | OCH₃ | OCH₃ | O | O |
| 1-207 | Br | H | COCH₂CH₃ | H | H | OCH₃ | OCH₃ | O | O |
| 1-208 | CH₃ | H | COCH₂CH₃ | H | H | OCH₃ | OCH₃ | O | O |
| 1-209 | H | H | COCH(CH₃)₂ | H | H | OCH₃ | OCH₃ | O | O |
| 1-210 | Cl | H | COCH(CH₃)₂ | H | H | OCH₃ | OCH₃ | O | O |
| 1-211 | Br | H | COCH(CH₃)₂ | H | H | OCH₃ | OCH₃ | O | O |
| 1-212 | CH₃ | H | COCH(CH₃)₂ | H | H | OCH₃ | OCH₃ | O | O |
| 1-213 | H | H | COC(CH₃)₃ | H | H | OCH₃ | OCH₃ | O | O |
| 1-214 | Cl | H | COC(CH₃)₃ | H | H | OCH₃ | OCH₃ | O | O |
| 1-215 | Br | H | COC(CH₃)₃ | H | H | OCH₃ | OCH₃ | O | O |
| 1-216 | CH₃ | H | COC(CH₃)₃ | H | H | OCH₃ | OCH₃ | O | O |
| 1-217 | H | H | COCH=CH₂ | H | H | OCH₃ | OCH₃ | O | S |
| 1-218 | Cl | H | COCH=CH₂ | H | H | OCH₃ | OCH₃ | O | S |
| 1-219 | Br | H | COCH=CH₂ | H | H | OCH₃ | OCH₃ | O | O |
| 1-220 | CH₃ | H | COCH=CH₂ | H | H | OCH₃ | OCH₃ | O | O |
| 1-221 | H | H | COC≡CH | H | H | OCH₃ | OCH₃ | O | O |
| 1-222 | Cl | H | COC≡CH | H | H | OCH₃ | OCH₃ | O | O |
| 1-223 | Br | H | COC≡CH | H | H | OCH₃ | OCH₃ | O | O |
| 1-224 | CH₃ | H | COC≡CH | H | H | OCH₃ | OCH₃ | O | O |
| 1-225 | H | H | COCH₂Cl | H | H | OCH₃ | OCH₃ | O | O |
| 1-226 | H | H | CH₂CH₂F | H | H | CH₃ | CH₃ | O | O |
| 1-227 | Cl | H | CH₂CH₂F | H | H | CH₃ | CH₃ | O | O |
| 1-228 | Br | H | CH₂CH₂F | H | H | CH₃ | CH₃ | O | O |
| 1-229 | CH₃ | H | CH₂CH₂F | H | H | CH₃ | CH₃ | O | O |
| 1-230 | CH₂CH₃ | H | CH₂CH₂F | H | H | CH₃ | CH₃ | O | O |
| 1-231 | OCH₃ | H | CH₂CH₂F | H | H | CH₃ | CH₃ | O | O |
| 1-232 | CF₃ | H | CH₂CH₂F | H | H | CH₃ | CH₃ | O | O |
| 1-233 | H | H | CH₂CF₃ | H | H | CH₃ | CH₃ | O | O |
| 1-234 | Cl | H | CH₂CF₃ | H | H | CH₃ | CH₃ | O | O |
| 1-235 | Br | H | CH₂CF₃ | H | H | CH₃ | CH₃ | O | O |
| 1-236 | CH₃ | H | CH₂CF₃ | H | H | CH₃ | CH₃ | O | O |
| 1-237 | CH₂CH₃ | H | CH₂CF₃ | H | H | CH₃ | CH₃ | O | O |
| 1-238 | OCH₃ | H | CH₂CF₃ | H | H | CH₃ | CH₃ | O | O |
| 1-239 | CF₃ | H | CH₂CF₃ | H | H | CH₃ | CH₃ | O | O |
| 1-240 | H | H | CH₂C≡CCH₃ | H | H | CH₃ | CH₃ | O | O |
| 1-241 | Cl | H | CH₂C≡CCH₃ | H | H | CH₃ | CH₃ | O | O |
| 1-242 | Br | H | CH₂C≡CCH₃ | H | H | CH₃ | CH₃ | O | O |
| 1-243 | CH₃ | H | CH₂C≡CCH₃ | H | H | CH₃ | CH₃ | O | O |
| 1-244 | CH₂CH₃ | H | CH₂C≡CCH₃ | H | H | CH₃ | CH₃ | O | O |
| 1-245 | OCH₃ | H | CH₂C≡CCH₃ | H | H | CH₃ | CH₃ | O | O |
| 1-246 | CF₃ | H | CH₂C≡CCH₃ | H | H | CH₃ | CH₃ | O | O |
| 1-247 | CN | H | CH₃ | H | H | CH₃ | CH₃ | O | O |
| 1-248 | CN | H | CH₂C≡CCH₃ | H | H | CH₃ | CH₃ | O | O |
| 1-249 | CN | H | CH₃ | H | H | CH₂C≡CCH₃ | CH₃ | O | O |
| 1-250 | CN | H | CH₂C≡CCH₃ | H | H | CH₂C≡CCH₃ | CH₃ | O | O |
| 1-251 | H | H | CH(CH₃)₂ | H | H | OCH₃ | OCH₃ | O | O |
| 1-252 | Cl | H | CH(CH₃)₂ | H | H | OCH₃ | OCH₃ | O | O |
| 1-253 | Br | H | CH(CH₃)₂ | H | H | OCH₃ | OCH₃ | O | O |
| 1-254 | CH₃ | H | CH(CH₃)₂ | H | H | OCH₃ | OCH₃ | O | O |
| 1-255 | CH₂CH₃ | H | CH(CH₃)₂ | H | H | OCH₃ | OCH₃ | O | O |
| 1-256 | OCH₃ | H | CH(CH₃)₂ | H | H | OCH₃ | OCH₃ | O | O |
| 1-257 | CF₃ | H | CH(CH₃)₂ | H | H | OCH₃ | OCH₃ | O | O |

In the table, "c-Pr" means cyclopropyl.

The compound of the following formula wherein R², R³, R⁴, R⁵, R⁷, R⁸, R¹¹, R¹² and R¹³ in the formula (1) are hydrogen atoms, and R¹, R⁶, R⁹, R¹⁰, R¹⁴, R¹⁵ R¹⁶ and R¹⁷ are the atom or group described in Table 2.

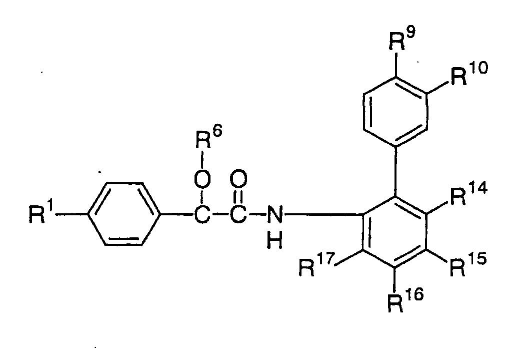

**Table 2**

| Number | R¹ | R⁶ | R⁹ | R¹⁰ | R¹⁴ | R¹⁵ | R¹⁶ | R¹⁷ |
|---|---|---|---|---|---|---|---|---|
| 2-001 | H | CH₃ | OC₂H₅ | OCH₃ | H | H | H | H |
| 2-002 | Cl | CH₃ | OC₂H₅ | OCH₃ | H | H | H | H |
| 2-003 | Br | CH₃ | OC₂H₅ | OCH₃ | H | H | H | H |
| 2-004 | CH₃ | CH₃ | OC₂H₅ | OCH₃ | H | H | H | H |
| 2-005 | CH₂CH₃ | CH₃ | OC₂H₅ | OCH₃ | H | H | H | H |
| 2-006 | OCH₃ | CH₃ | OC₂H₅ | OCH₃ | H | H | H | H |
| 2-007 | CF₃ | CH₃ | OC₂H₅ | OCH₃ | H | H | H | H |
| 2-008 | H | CH₂C≡CH | OC₂H₅ | OCH₃ | H | H | H | H |
| 2-009 | Cl | CH₂C≡CH | OC₂H₅ | OCH₃ | H | H | H | H |
| 2-010 | Br | CH₂C≡CH | OC₂H₅ | OCH₃ | H | H | H | H |
| 2-011 | CH₃ | CH₂C≡CH | OC₂H₅ | OCH₃ | H | H | H | H |
| 2-012 | CH₂CH₃ | CH₂C≡CH | OC₂H₅ | OCH₃ | H | H | H | H |
| 2-013 | OCH₃ | CH₂C≡CH | OC₂H₅ | OCH₃ | H | H | H | H |
| 2-014 | CF₃ | CH₂C≡CH | OC₂H₅ | OCH₃ | H | H | H | H |
| 2-015 | H | CH₃ | OCH₂C≡CH | OCH₃ | H | H | H | H |
| 2-016 | Cl | CH₃ | OCH₂C≡CH | OCH₃ | H | H | H | H |
| 2-017 | Br | CH₃ | OCH₂C≡CH | OCH₃ | H | H | H | H |
| 2-018 | CH₃ | CH₃ | OCH₂C≡CH | OCH₃ | H | H | H | H |
| 2-019 | CH₂CH₃ | CH₃ | OCH₂C≡CH | OCH₃ | H | H | H | H |
| 2-020 | OCH₃ | CH₃ | OCH₂C≡CH | OCH₃ | H | H | H | H |
| 2-021 | CF₃ | CH₃ | OCH₂C≡CH | OCH₃ | H | H | H | H |
| 2-022 | H | CH₂C≡CH | OCH₂C≡CH | OCH₃ | H | H | H | H |
| 2-023 | Cl | CH₂C≡CH | OCH₂C≡CH | OCH₃ | H | H | H | H |
| 2-024 | Br | CH₂C≡CH | OCH₂C≡CH | OCH₃ | H | H | H | H |
| 2-025 | CH₃ | CH₂C≡CH | OCH₂C≡CH | OCH₃ | H | H | H | H |
| 2-026 | CH₂CH₃ | CH₂C≡CH | OCH₂C≡CH | OCH₃ | H | H | H | H |
| 2-027 | OCH₃ | CH₂C≡CH | OCH₂C≡CH | OCH₃ | H | H | H | H |
| 2-028 | CF₃ | CH₂C≡CH | OCH₂C≡CH | OCH₃ | H | H | H | H |
| 2-029 | H | CH₃ | OCH₂CN | OCH₃ | H | H | H | H |
| 2-030 | Cl | CH₃ | OCH₂CN | OCH₃ | H | H | H | H |
| 2-031 | Br | CH₃ | OCH₂CN | OCH₃ | H | H | H | H |
| 2-032 | CH₃ | CH₃ | OCH₂CN | OCH₃ | H | H | H | H |
| 2-033 | CH₂CH₃ | CH₃ | OCH₂CN | OCH₃ | H | H | H | H |
| 2-034 | OCH₃ | CH₃ | OCH₂CN | OCH₃ | H | H | H | H |
| 2-035 | CF₃ | CH₃ | OCH₂CN | OCH₃ | H | H | H | H |
| 2-036 | H | CH₂C≡CH | OCH₂CN | OCH₃ | H | H | H | H |
| 2-037 | Cl | CH₂C≡CH | OCH₂CN | OCH₃ | H | H | H | H |
| 2-038 | Br | CH₂C≡CH | OCH₂CN | OCH₃ | H | H | H | H |
| 2-039 | CH₃ | CH₂C≡CH | OCH₂CN | OCH₃ | H | H | H | H |
| 2-040 | CH₂CH₃ | CH₂C≡CH | OCH₂CN | OCH₃ | H | H | H | H |
| 2-041 | OCH₃ | CH₂C≡CH | OCH₂CN | OCH₃ | H | H | H | H |
| 2-042 | CF₃ | CH₂C≡CH | OCH₂CN | OCH₃ | H | H | H | H |
| 2-043 | Cl | CH₃ | OCH₃ | OCH₃ | CH₃ | H | H | H |
| 2-044 | CH₃ | CH₃ | OCH₃ | OCH₃ | CH₃ | H | H | H |
| 2-045 | Cl | CH₂C≡CH | OCH₃ | OCH₃ | CH₃ | H | H | H |
| 2-046 | CH₃ | CH₂C≡CH | OCH₃ | OCH₃ | CH₃ | H | H | H |
| 2-047 | Cl | CH₃ | OCH₃ | OCH₃ | Cl | H | H | H |
| 2-048 | CH₃ | CH₃ | OCH₃ | OCH₃ | Cl | H | H | H |
| 2-049 | Cl | CH₂C≡CH | OCH₃ | OCH₃ | Cl | H | H | H |
| 2-050 | CH₃ | CH₂C≡CH | OCH₃ | OCH₃ | Cl | H | H | H |
| 2-051 | Cl | CH₃ | OCH₃ | OCH₃ | OCH₃ | H | H | H |
| 2-052 | CH₃ | CH₃ | OCH₃ | OCH₃ | OCH₃ | H | H | H |
| 2-053 | Cl | CH₂C≡CH | OCH₃ | OCH₃ | OCH₃ | H | H | H |
| 2-054 | CH₃ | CH₂C≡CH | OCH₃ | OCH₃ | OCH₃ | H | H | H |
| 2-055 | Cl | CH₃ | OCH₃ | OCH₃ | CN | H | H | H |
| 2-056 | CH₃ | CH₃ | OCH₃ | OCH₃ | CN | H | H | H |
| 2-057 | Cl | CH₂C≡CH | OCH₃ | OCH₃ | CN | H | H | H |
| 2-058 | CH₃ | CH₂C≡CH | OCH₃ | OCH₃ | CN | H | H | H |
| 2-059 | Cl | CH₃ | OCH₃ | OCH₃ | H | CH₃ | H | H |
| 2-060 | CH₃ | CH₃ | OCH₃ | OCH₃ | H | CH₃ | H | H |
| 2-061 | Cl | CH₂C≡CH | OCH₃ | OCH₃ | H | CH₃ | H | H |
| 2-062 | CH₃ | CH₂C≡CH | OCH₃ | OCH₃ | H | CH₃ | H | H |
| 2-063 | Cl | CH₃ | OCH₃ | OCH₃ | H | Cl | H | H |
| 2-064 | CH₃ | CH₃ | OCH₃ | OCH₃ | H | Cl | H | H |
| 2-065 | Cl | CH₂C≡CH | OCH₃ | OCH₃ | H | Cl | H | H |
| 2-066 | CH₃ | CH₂C≡CH | OCH₃ | OCH₃ | H | Cl | H | H |
| 2-067 | Cl | CH₃ | OCH₃ | OCH₃ | H | OCH₃ | H | H |
| 2-068 | CH₃ | CH₃ | OCH₃ | OCH₃ | H | OCH₃ | H | H |
| 2-069 | Cl | CH₂C≡CH | OCH₃ | OCH₃ | H | OCH₃ | H | H |
| 2-070 | CH₃ | CH₂C≡CH | OCH₃ | OCH₃ | H | OCH₃ | H | H |
| 2-071 | Cl | CH₃ | OCH₃ | OCH₃ | H | CN | H | H |
| 2-072 | CH₃ | CH₃ | OCH₃ | OCH₃ | H | CN | H | H |
| 2-073 | Cl | CH₂C≡CH | OCH₃ | OCH₃ | H | CN | H | H |
| 2-074 | CH₃ | CH₂C≡CH | OCH₃ | OCH₃ | H | CN | H | H |
| 2-075 | Cl | CH₃ | OCH₃ | OCH₃ | H | H | CH₃ | H |
| 2-076 | CH₃ | CH₃ | OCH₃ | OCH₃ | H | H | CH₃ | H |
| 2-077 | Cl | CH₂C≡CH | OCH₃ | OCH₃ | H | H | CH₃ | H |
| 2-078 | CH₃ | CH₂C=CH | OCH₃ | OCH₃ | H | H | CH₃ | H |
| 2-079 | Cl | CH₃ | OCH₃ | OCH₃ | H | H | Cl | H |
| 2-080 | CH₃ | CH₃ | OCH₃ | OCH₃ | H | H | Cl | H |
| 2-081 | Cl | CH₂C≡CH | OCH₃ | OCH₃ | H | H | Cl | H |
| 2-082 | CH₃ | CH₂C≡CH | OCH₃ | OCH₃ | H | H | Cl | H |
| 2-083 | Cl | CH₃ | OCH₃ | OCH₃ | H | H | OCH₃ | H |
| 2-084 | CH₃ | CH₃ | OCH₃ | OCH₃ | H | H | OCH₃ | H |
| 2-085 | Cl | CH₂C≡CH | OCH₃ | OCH₃ | H | H | OCH₃ | H |
| 2-086 | CH₃ | CH₂C≡CH | OCH₃ | OCH₃ | H | H | OCH₃ | H |
| 2-087 | Cl | CH₃ | OCH₃ | OCH₃ | H | H | CN | H |
| 2-088 | CH₃ | CH₃ | OCH₃ | OCH₃ | H | H | CN | H |
| 2-089 | Cl | CH₂C≡CH | OCH₃ | OCH₃ | H | H | CN | H |
| 2-090 | CH₃ | CH₂C≡CH | OCH₃ | OCH₃ | H | H | CN | H |
| 2-091 | Cl | CH₃ | OCH₃ | OCH₃ | H | H | H | CH₃ |
| 2-092 | CH₃ | CH₃ | OCH₃ | OCH₃ | H | H | H | CH₃ |
| 2-093 | Cl | CH₂C≡CH | OCH₃ | OCH₃ | H | H | H | CH₃ |
| 2-094 | CH₃ | CH₂C≡CH | OCH₃ | OCH₃ | H | H | H | CH₃ |
| 2-095 | Cl | CH₃ | OCH₃ | OCH₃ | H | H | H | Cl |
| 2-096 | CH₃ | CH₃ | OCH₃ | OCH₃ | H | H | H | Cl |
| 2-097 | Cl | CH₂C≡CH | OCH₃ | OCH₃ | H | H | H | Cl |
| 2-098 | CH₃ | CH₂C≡CH | OCH₃ | OCH₃ | H | H | H | Cl |
| 2-099 | Cl | CH₃ | OCH₃ | OCH₃ | H | H | H | OCH₃ |
| 2-100 | CH₃ | CH₃ | OCH₃ | OCH₃ | H | H | H | OCH₃ |
| 2-101 | Cl | CH₂C≡CH | OCH₃ | OCH₃ | H | H | H | OCH₃ |
| 2-102 | CH₃ | CH₃ | OCH₃ | OCH₃ | H | H | H | CN |
| 2-103 | Cl | CH₂C≡CH | OCH₃ | OCH₃ | H | H | H | CN |
| 2-104 | CH₃ | CH₂C≡CH | OCH₃ | OCH₃ | H | H | H | CN |
| 2-105 | Cl | CH₂C≡CH | OCH₃ | OCH₃ | Cl | Cl | H | H |
| 2-106 | CH₃ | CH₂C≡CH | OCH₃ | OCH₃ | Cl | Cl | H | H |
| 2-107 | Cl | CH₂C≡CH | OCH₃ | OCH₃ | Cl | H | Cl | H |
| 2-108 | CH₃ | CH₂C≡CH | OCH₃ | OCH₃ | Cl | H | Cl | H |
| 2-109 | Cl | CH₂C≡CH | OCH₃ | OCH₃ | Cl | H | H | Cl |
| 2-110 | CH₃ | CH₂C≡CH | OCH₃ | OCH₃ | Cl | H | H | Cl |
| 2-111 | Cl | CH₂C≡CH | OCH₃ | OCH₃ | H | Cl | Cl | H |
| 2-112 | CH₃ | CH₂C≡CH | OCH₃ | OCH₃ | H | Cl | Cl | H |
| 2-113 | Cl | CH₂C≡CH | OCH₃ | OCH₃ | H | H | Cl | Cl |
| 2-114 | CH₃ | CH₂C≡CH | OCH₃ | OCH₃ | H | H | Cl | Cl |
| 2-115 | Cl | CH₂C≡CH | OCH₃ | OCH₃ | CH₃ | CH₃ | H | H |
| 2-116 | CH₃ | CH₂C≡CH | OCH₃ | OCH₃ | CH₃ | CH₃ | H | H |
| 2-117 | Cl | CH₂C≡CH | OCH₃ | OCH₃ | CH₃ | H | CH₃ | H |
| 2-118 | CH₃ | CH₂C≡CH | OCH₃ | OCH₃ | CH₃ | H | CH₃ | H |
| 2-119 | Cl | CH₂C≡CH | OCH₃ | OCH₃ | CH₃ | H | H | CH₃ |
| 2-120 | CH₃ | CH₂C≡CH | OCH₃ | OCH₃ | CH₃ | H | H | CH₃ |
| 2-121 | Cl | CH₂C≡CH | OCH₃ | OCH₃ | H | CH₃ | CH₃ | H |
| 2-122 | CH₃ | CH₂C≡CH | OCH₃ | OCH₃ | H | CH₃ | CH₃ | H |
| 2-123 | Cl | CH₂C≡CH | OCH₃ | OCH₃ | H | H | CH₃ | CH₃ |
| 2-124 | CH₃ | CH₂C≡CH | OCH₃ | OCH₃ | H | H | CH₃ | CH₃ |
| 2-125 | Cl | CH₂C≡CH | OCH₃ | OCH₃ | CH₃ | F | H | H |
| 2-126 | CH₃ | CH₂C≡CH | OCH₃ | OCH₃ | CH₃ | F | H | H |
| 2-127 | Cl | CH₂C≡CH | OCH₃ | OCH₃ | Cl | H | F | H |
| 2-128 | CH₃ | CH₂C≡CH | OCH₃ | OCH₃ | Cl | H | F | H |
| 2-129 | Cl | CH₂C≡CH | OCH₃ | OCH₃ | OCH₃ | H | H | Cl |
| 2-130 | CH₃ | CH₂C≡CH | OCH₃ | OCH₃ | OCH₃ | H | H | Cl |
| 2-131 | Cl | CH₂C≡CH | OCH₃ | OCH₃ | H | F | Cl | H |
| 2-132 | CH₃ | CH₂C≡CH | OCH₃ | OCH₃ | H | F | Cl | H |
| 2-133 | Cl | CH₂C≡CH | OCH₃ | OCH₃ | H | H | OCH₃ | CH₃ |
| 2-134 | CH₃ | CH₂C≡CH | OCH₃ | OCH₃ | H | H | OCH₃ | CH₃ |
| 2-133 | Cl | CH₂C≡CH | OCH₃ | OCH₃ | CF₃ | H | H | H |
| 2-134 | CH₃ | CH₂C≡CH | OCH₃ | OCH₃ | CF₃ | H | H | H |
| 2-137 | Cl | CH₂C≡CH | OCH₃ | OCH₃ | H | OCF₃ | H | H |
| 2-138 | CH₃ | CH₂C≡CH | OCH₃ | OCH₃ | H | OCF₃ | H | H |
| 2-139 | Cl | CH₂C≡CH | OCH₃ | OCH₃ | H | H | c-Pr | H |
| 2-140 | CH₃ | CH₂C≡CH | OCH₃ | OCH₃ | H | H | c-Pr | H |
| 2-141 | Cl | CH₂C≡CH | OCH₃ | OCH₃ | H | H | H | SCH₃ |
| 2-142 | CH₃ | CH₂C≡CH | OCH₃ | OCH₃ | H | H | H | SCH₃ |

In the table, "c-Pr" means cyclopropyl.

Next formulation examples are shown. Parts represent parts by weight. The present compounds are described with the above-mentioned numbers.

### Formulation Example 1

Fifty parts of each of the present compounds 1-001 to 1-257 and 2-001 to 1-142, 3 parts of calcium ligninsulfonate, 2 parts of magnesium laurylsulfate and 45 parts of synthetic hydrated silica are pulverized and mixed well to give wettable powders of each compound.

### Formulation Example 2

Twenty parts of each of the present compounds 1-001 to 1-257 and 2-001 to 1-142 and 1.5 parts of sorbitan trioleate are mixed with 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol are added to give a flowable of each compound.

### Formulation Example 3

Two parts of each of the present compounds 1-001 to 1-257 and 2-001 to 1-142, 88 parts of kaolin clay and 10 parts of talc are pulverized and mixed well to give dusts of each compound.

### Formulation Example 4

Five parts of each of the present compounds 1-001 to 1-257 and 2-001 to 1-142, 14 parts of polyoxyethylenestyryl phenyl ether, 6 parts of calcium dodecylbenzenesulfonate and 75 parts of xylene are mixed well to give emulsifiable concentrates of each compound.

### Formulation Example 5

Two parts of each of the present compounds 1-001 to 1-257 and 2-001 to 1-142, 1 part of synthetic hydrated silica, 2 parts of calcium ligninsulfonate, 30 parts of bentonite and 65 parts of kaolin clay are pulverized and mixed well, and water is added thereto and kneeded, granulated and dried to give granules of each compound.

### Formulation Example 6

Ten parts of each of the present compounds 1-001 to 1-257 and 2-001 to 1-142, 35 parts of white carbon containing 50% of ammonium polyoxyethylenealkyl ether sulfate and 55 parts of water are mixed and wet pulverized finely to give a formulation of each compound.

Next, it is shown that a present compounds are useful for controlling plant diseases. The present compounds are described with the above-mentioned numbers.

For comparison, a compound of number 4.1 described in European Patent Published Application 0545099, namely N- biphenyl-2-yl-2-methylbenzamide (hereinafter, refered to as the comparative compound A), was also subjected to the examination.

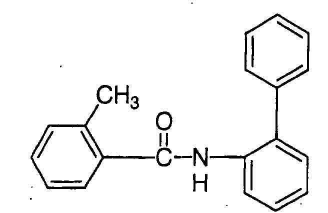

Additionally, the control effect was evaluated by visually observing the area of a lesion on a sample plant in investigation and comparing the area of a lesion on a non-treatment plant and the area of a lesion on a treated plant with the present compound.

### Test Example 1

Sand loam was compacted in a plastic pot, and a tomato (variety: Ponterosa) was seeded and grown in a green house for 20 days. The present compounds 1-006, 1-058, 1-059, 1-060, 1-063, 1-064, 1-080, 1-098, 1-111, 1-202, 1-210, 1-227, 1-234, 1-252, 2-025, 2-046, 2-062, 2-078 and the comparative compound A were formulated according to Formulation Example 6, then, diluted with water to provide given concentration of 500ppm, and these were sprayed onto stems and leaves so as to give sufficient adhesion on the surface of the tomato leaves. After spraying, the liquid on the stem was air-dried, and a suspension of zoosporangiua of Phytophthora infestans was inoculated by spraying. After inoculation, the plant was first grown for one day at 23°C under high humidity, then further grown for 4 days in the green house.

The areas of a lesion of the plants were visually obserbed. The lesion areas on plants on the plants treated with the present compound 1-006, 1-058, 1-059, 1-060, 1-063, 1-064, 1-080, 1-098, 1-111, 1-202, 1-210, 1-227, 1-234, 1-252, 2-025, 2-046, 2-062 and 2-078 were not more than 10% of the lesion area on a non-treatment plant. The lesion areas on plants on the plants treated with the comparative compound A was in a range of 76 to 100% of the lesion area on a non-treatment plant.

### Test Example 2

Sand loam was compacted in a plastic pot, and a grape (variety: Berry A) was seeded and grown in a green house for 40 days. The present compounds 1-003, 1-006, 1-058, 1-059, 1-060, 1-063, 1-064, 1-080, 1-098, 1-111, 1-210, 1-214, 1-227, 1-252, 2-025, 2-046, 2-062 and 2-078 were formulated according to Formulation Example 6, then, diluted with water to provide given concentration of 200ppm, and these were sprayed onto stems and leaves so as to give sufficient adhesion on the surface of grape leaves. After spraying, the liquid on the stem was air-dried, and a suspension of zoosporangiua of Plasmopara viticola was inoculated by spraying. After inoculation, the plant was first grown for one day at 23°C under high humidity, then further grown for 6 days in the green house, then the control effect was checked.

The areas of a lesion of the plants were visually obserbed. The lesion areas on plants on the plants treated with the present compound 1-003, 1-006, 1-058, 1-059, 1-060, 1-063, 1-064, 1-080, 1-098, 1-111, 1-210, 1-214, 1-227, 1-252, 2-025, 2-046, 2-062 and 2-078 were not more than 10% of the lesion area on a non-treatment plant.

### Industrial Applicability

By using the present compound, plant diseases can be controlled.

## Claims

1. A N-biphenylamide compound of the formula (1): 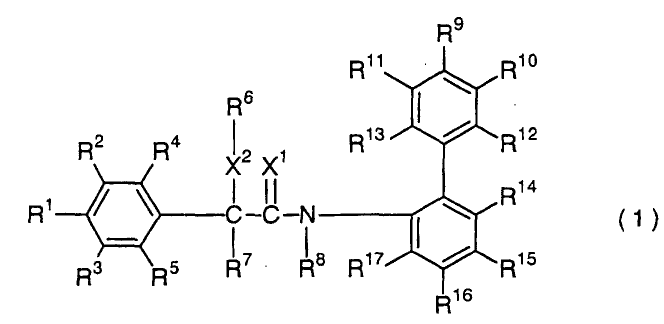 wherein R¹, R², R³, R⁴ and R⁵ independently represent a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkoxy, tri(C1-C6 alkyl)silyl or cyano,
R⁶ represents C1-C6 alkyl, C1-C6 haloalkyl, C3-C6 alkenyl, C3-C6 haloalkenyl, C3-C6 alkynyl, C3-C6 haloalkynyl, (C1-C6 alkyl)carbonyl, (C1-C6 haloalkyl)carbonyl, (C2-C6 alkenyl)carbonyl, (C2-C6 haloalkenyl)carbonyl, (C2-C6 alkynyl)carbonyl or (C2-C6 haloalkynyl)carbonyl,
R⁷ represents a hydrogen atom or C1-C3 alkyl,
R⁸ represents a hydrogen atom or C1-C3 alkyl,
R⁹ and R¹⁰ independently represents a halogen atom, C1-C6 alkyl, C1-C6 haloalkyl, C2-C6 alkenyl, C2-C6 haloalkenyl, C2-C6 alkynyl, C2-C6 haloalkynyl, cyano C1-C6 alkyl, C1-C6 alkoxy, C1-C6 haloalkoxy, C3-C6 alkenyloxy, C3-C6 haloalkenyloxy, C3-C6 alkynyloxy, C3-C6 haloalkynyloxy, cyano C1-C6 alkoxy, C1-C6 alkylthio, C1-C6 haloalkylthio, C3-C6 alkenylthio, C3-C6 alkynylthio, C3-C6 cycloalkyl, C3-C6 cycloalkoxy, (C3-C6 cycloalkyl) C1-C3 alkoxy, C2-C6 alkoxyalkyl, tri(C1-C6 alkyl)silyl or cyano,
R¹¹, R¹² and R¹³ independently represent a hydrogen atom, halogen atom or C1-C3 alkyl,
R¹⁴, R¹⁵, R¹⁶ and R¹⁷ independently is a hydrogen atom, halogen atom, C1-C6 alkyl, C1-C6 haloalkyl, C2-C6 alkenyl, C2-C6 haloalkenyl, C2-C6 alkynyl, C2-C6 haloalkynyl, C1-C6 alkoxy, C1-C6 haloalkoxy, C3-C6 alkenyloxy, C3-C6 haloalkenyloxy, C3-C6 alkynyloxy, C3-C6 haloalkynyloxy, C1-C6 alkylthio, C1-C6 haloalkylthio, C3-C6 cycloalkyl, C3-C6 cycloalkyloxy, tri(C1-C6 alkyl)silyl or cyano,
X¹ represents an oxygen atom or sulfur atom,
X² represents an oxygen atom or sulfur atom.

2. The N-biphenylamide compound according to cliam 1; wherein X¹ is an oxygen atom in the formula (1).

3. The N-biphenylamide compound according to claim 1 or 2; wherein X² is an oxygen atom in the formula (1).

4. The N-biphenylamide compound according to any of claim 1 to 3; wherein R⁷ is a hydrogen atom in the formula (1).

5. The N-biphenylamide compound according to claim 1; wherein R⁹ is C1-C6 alkoxy in the formula (1).

6. The N-biphenylamide compound according to claim 1; wherein X¹ and X² are oxygen atoms, R⁷ is a hydrogen atom, and R⁹ is C1-C6 alkoxy in the formula (1).

7. The N-biphenylamide compound according to claim 1; wherein R¹⁰ is C1-C6 alkoxy in the formula (1).

8. The N-biphenylamide compound according to claim 1; wherein X¹ and X² are oxygen atoms, R⁷ is a hydrogen atom, R¹⁰ is C1-C6 alkoxy in the formula (1).

9. The N-biphenylamide compound according to claim 1; R⁹ and R¹⁰ are C1-C6 alkoxy in the formula (1).

10. The N-biphenylamide compound according to claim 1; X¹ and X² are oxygen atoms, R⁷ is a hydrogen atom, and R⁹ and R¹⁰ are C1-C6 alkoxy in the formula (1).

11. A fungicidal composition comprising the N-biphenylamide compound described in claim 1 as an active ingredient.

12. A method for controlling plant diseases comprising a step of applying the N-biphenylamide compound described in claim 1 to a plant or the soil.

13. Use of the N-biphenylamide compound described in claim 1 as an active ingredient of fungicidal composition.
